# EUROPEAN PATENT APPLICATION

(11) **EP 4 233 573 A2**
(43) Date of publication of application: **30.08.2023**
(21) Application number: 23165253.8
(22) Date of filing: 19.11.2019
(51) Int. Cl.: A24D 1/00, A24D 1/02, A24D 1/20, A24D 1/22, A24F 40/20, A24F 40/30, A24F 40/40, A61M 11/04, A61M 15/06, A61M 16/00

(54) **OVERWRAP MATERIAL CONTAINING AEROSOL FORMER FOR AEROSOL SOURCE MEMBER**

(30) Priority: 20.11.2018 US 201816197077
(62) Divisional of application: 19809639.8
(71) Applicant: R. J. Reynolds Tobacco Company, Winston-Salem, North Carolina 27101-3804 (US)
(72) Inventor: SEBASTIAN, Andries D., Winston-Salem, NC 27103 (US); CONNER, Billy T., Clemmons, NC 27012 (US); HEJAZI, Vahid, Concord, NC 28027 (US); MUA, John-Paul, Advance, NC 27006 (US); MONSALUD, Luis, Kernersville, NC 27284 (US); SEARS, Stephen B., Siler City, NC 27344 (US); COLE, S. Keith, Advance, NC 27006 (US)
(74) Representative: Dehns

(57) **Abstract**

The present disclosure provides an aerosol source member, and an overwrap material configured to substantially surround a substrate portion of an aerosol source member. In an example implementation, the aerosol source member may comprise a substrate portion that includes a substrate material configured to generate a primary inhalable substance upon the application of heat thereupon, the substrate portion defining an outer surface, and an overwrap material configured to substantially surround the outer surface of the substrate portion, wherein the overwrap material comprises a plurality of layers, and wherein at least one of the layers of the overwrap material contains an aerosol generating component.

## Description

### Cross-Reference to Related Applications

This application claims priority to, and the benefit of, U.S. Patent Application No. 16/197,077, titled Overwrap Material Containing Aerosol Former for Aerosol Source Member, filed on November 20, 2018, which is incorporated herein in its entirety by reference.

### Field of the Disclosure

The present disclosure relates to aerosol source members and uses thereof for yielding tobacco components or other materials in inhalable form. More particularly, the present disclosure relates to overwrap materials and aerosol source members that include overwrap materials for use with aerosol delivery devices and systems, such as smoking articles, that utilize electrically-generated heat or combustible carbon-based ignition sources to heat a tobacco or non-tobacco material, preferably without significant combustion, in order to provide an inhalable substance in the form of an aerosol for human consumption.

### Description of Related Art

Many smoking articles have been proposed through the years as improvements upon, or alternatives to, smoking products based upon combusting tobacco. Some example alternatives have included devices wherein a solid or liquid fuel is combusted to transfer heat to tobacco or wherein a chemical reaction is used to provide such heat source. Additional example alternatives use electrical energy to heat tobacco and/or other aerosol generating substrate materials, such as described in U.S. Patent No. 9,078,473 to Worm et al., which is incorporated herein by reference in its entirety.

The point of the improvements or alternatives to smoking articles typically has been to provide the sensations associated with cigarette, cigar, or pipe smoking, without delivering considerable quantities of incomplete combustion and pyrolysis products. To this end, there have been proposed numerous smoking products, flavor generators, and medicinal inhalers which utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 7,726,320 to Robinson et al.; and U.S. Pat. App. Pub. Nos. 2013/0255702 to Griffith, Jr. et al.; and 2014/0096781 to Sears et al., which are incorporated herein by reference in their entireties. See also, for example, the various types of smoking articles, aerosol delivery devices and electrically powered heat generating sources referenced by brand name and commercial source in U.S. Pat. App. Pub. No. 2015/0220232 to Bless et al., which is incorporated herein by reference in its entirety. Additional types of smoking articles, aerosol delivery devices and electrically powered heat generating sources referenced by brand name and commercial source are listed in U.S. Pat. App. Pub. No. 2015/0245659 to DePiano et al., which is also incorporated herein by reference in its entirety. Other representative cigarettes or smoking articles that have been described and, in some instances, been made commercially available include those described in U.S. Pat. No. 4,735,217 to Gerth et al.; U.S. Pat Nos. 4,922,901, 4,947,874, and 4,947,875 to Brooks et al.; U.S. Pat. No. 5,060,671 to Counts et al.; U.S. Pat. No. 5,249,586 to Morgan et al.; U.S. Pat. No. 5,388,594 to Counts et al.; U.S. Pat. No. 5,666,977 to Higgins et al.; U.S. Pat. No. 6,053,176 to Adams et al.; U.S. 6,164,287 to White; U.S. Pat No. 6,196,218 to Voges; U.S. Pat. No. 6,810,883 to Felter et al.; U.S. Pat. No. 6,854,461 to Nichols; U.S. Pat. No. 7,832,410 to Hon; U.S. Pat. No. 7,513,253 to Kobayashi; U.S. Pat. No. 7,726,320 to Robinson et al.; U.S. Pat. No. 7,896,006 to Hamano; U.S. Pat. No. 6,772,756 to Shayan; U.S. Pat. Pub. No. 2009/0095311 to Hon; U.S. Pat. Pub. Nos. 2006/0196518, 2009/0126745, and 2009/0188490 to Hon; U.S. Pat. Pub. No. 2009/0272379 to Thorens et al.; U.S. Pat. Pub. Nos. 2009/0260641 and 2009/0260642 to Monsees et al.; U.S. Pat. Pub. Nos. 2008/0149118 and 2010/0024834 to Oglesby et al.; U.S. Pat. Pub. No. 2010/0307518 to Wang; and WO 2010/091593 to Hon, which are incorporated herein by reference in their entireties.

Representative products that resemble many of the attributes of traditional types of cigarettes, cigars or pipes have been marketed as ACCORD^{®} by Philip Morris Incorporated; ALPHA^{™}, JOYE 510^{™} and M4^{™} by InnoVapor LLC; CIRRUS^{™} and FLING^{™} by White Cloud Cigarettes; BLU^{™} by Fontem Ventures B.V.; COHITA^{™}, COLIBRI^{™}, ELITE CLASSIC^{™}, MAGNUM^{™}, PHANTOM^{™} and SENSE^{™} by EPUFFER^{®} International Inc.; DUOPRO^{™}, STORM^{™} and VAPORKING^{®} by Electronic Cigarettes, Inc.; EGAR^{™} by Egar Australia; eGo-C^{™} and eGo-T^{™} by Joyetech; ELUSION^{™} by Elusion UK Ltd; EONSMOKE^{®} by Eonsmoke LLC; FIN^{™} by FIN Branding Group, LLC; SMOKE^{®} by Green Smoke Inc. USA; GREENARETTE^{™} by Greenarette LLC; HALLIGAN^{™}, HENDU^{™}, JET^{™}, MAXXQ^{™}, PINK^{™} and PITBULL^{™} by SMOKE STIK^{®}; HEATBAR^{™} by Philip Morris International, Inc.; HYDRO IMPERIAL^{™} and LXE^{™} from Crown7; LOGIC^{™} and THE CUBAN^{™} by LOGIC Technology; LUCI^{®} by Luciano Smokes Inc.; METRO^{®} by Nicotek, LLC; NJOY^{®} and ONEJOY^{™} by Sottera, Inc.; NO. 7^{™} by SS Choice LLC; PREMIUM ELECTRONIC CIGARETTE^{™} by PremiumEstore LLC; RAPP E-MYSTICK^{™} by Ruyan America, Inc.; RED DRAGON^{™} by Red Dragon Products, LLC; RUYAN^{®} by Ruyan Group (Holdings) Ltd.; SF^{®} by Smoker Friendly International, LLC; GREEN SMART SMOKER^{®} by The Smart Smoking Electronic Cigarette Company Ltd.; SMOKE ASSIST^{®} by Coastline Products LLC; SMOKING EVERYWHERE^{®} by Smoking Everywhere, Inc.; V2CIGS^{™} by VMR Products LLC; VAPOR NINE^{™} by VaporNine LLC; VAPOR4LIFE^{®} by Vapor 4 Life, Inc.; VEPPO^{™} by E-CigaretteDirect, LLC; VUSE^{®} by R. J. Reynolds Vapor Company; Mistic Menthol product by Mistic Ecigs; and the Vype product by CN Creative Ltd; IQOS^{™} by Philip Morris International; and GLO^{™} by British American Tobacco. Yet other electrically powered aerosol delivery devices, and in particular those devices that have been characterized as so-called electronic cigarettes, have been marketed under the tradenames COOLER VISIONS^{™}; DIRECT E-CIG^{™}; DRAGONFLY^{™}; EMIST^{™}. EVERSMOKE^{™}; GAMUCCI^{®}; HYBRID FLAME^{™}; KNIGHT STICKS^{™}; ROYAL BLUES^{™}; SMOKETIP^{®}; and SOUTH BEACH SMOKE^{™}.

Articles that produce the taste and sensation of smoking by electrically heating tobacco, tobacco derived materials, or other plant derived materials have suffered from inconsistent performance characteristics. For example, some articles have suffered from inconsistent release of flavors or other inhalable materials. Accordingly, it can be desirable to provide a smoking article that can provide the sensations of cigarette, cigar, or pipe smoking, that does so without combusting the substrate material, and that does so with advantageous performance characteristics.

### BRIEF SUMMARY

In various implementations, the present disclosure provides an aerosol source member and an overwrap material configured to substantially surround an outer surface of a substrate portion of an aerosol source member.

**Example Implementation 1:** An aerosol source member comprising a substrate portion that includes a substrate material configured to generate a primary inhalable substance upon the application of heat thereupon, the substrate portion defining an outer surface, and an overwrap material configured to substantially surround the outer surface of the substrate portion, wherein the overwrap material comprises a plurality of layers, and wherein at least one of the layers of the overwrap material contains an aerosol generating component.

**Example Implementation 2:** The aerosol source member of any preceding example implementation, or any combination of any preceding example implementations, wherein the aerosol generating component contained in the overwrap material has an aerosol former loading of approximately 2% - 20%.

**Example Implementation 3:** The aerosol source member of any preceding example implementation, or any combination of any preceding example implementations, wherein the aerosol generating component contained in the overwrap material has an aerosol former loading of approximately 20% - 40%.

**Example Implementation 4:** The aerosol source member of any preceding example implementation, or any combination of any preceding example implementations, wherein the overwrap material comprises a three layer laminate that includes an outer layer, an inner layer positioned proximate an outer surface of the substrate material, and an intermediate layer positioned between the outer layer and the inner layer.

**Example Implementation 5:** The aerosol source member of any preceding example implementation, or any combination of any preceding example implementations, wherein the outer layer comprises a paper material, the intermediate layer comprises a foil material, and the inner layer comprises an aerosol generating material.

**Example Implementation 6:** The aerosol source member of any preceding example implementation, or any combination of any preceding example implementations, wherein the aerosol generating material comprises a cast sheet containing a fibrous material.

**Example Implementation 7:** The aerosol source member of any preceding example implementation, or any combination of any preceding example implementations, wherein the aerosol generating material comprises a reconstituted tobacco sheet.

**Example Implementation 8:** The aerosol source member of any preceding example implementation, or any combination of any preceding example implementations, wherein the overwrap material is configured to produce an initial inhalable substance prior to production of a primary inhalable substance from the substrate material.

**Example Implementation 9:** The aerosol source member of any preceding example implementation, or any combination of any preceding example implementations, wherein the overwrap material is configured to produce a secondary inhalable substance after production of a primary inhalable substance from the substrate material.

**Example Implementation 10:** The aerosol source member of any preceding example implementation, or any combination of any preceding example implementations, wherein the overwrap material is configured to produce the initial inhalable substance in response to application of heat from an electrically generated heat source.

**Example Implementation 11:** The aerosol source member of any preceding example implementation, or any combination of any preceding example implementations, wherein the overwrap material is configured to produce the initial inhalable substance in response to application of heat from a combustible carbon-based heat source.

**Example Implementation 12:** An overwrap material configured to substantially surround an outer surface of a substrate portion of an aerosol source member, the overwrap comprising a plurality of layers, wherein at least one of the layers of the overwrap material contains an aerosol generating component.

**Example Implementation 13:** The overwrap material of any preceding example implementation, or any combination of any preceding example implementations, wherein the aerosol generating component contained in the overwrap material has an aerosol former loading of approximately 2% - 20%.

**Example Implementation 14:** The overwrap material of any preceding example implementation, or any combination of any preceding example implementations, wherein the aerosol generating component contained in the overwrap material has an aerosol former loading of approximately 20% - 40%.

**Example Implementation 15:** The overwrap material of any preceding example implementation, or any combination of any preceding example implementations, wherein the overwrap material comprises a three layer laminate that includes an outer layer, an inner layer positioned proximate an outer surface of the substrate material, and an intermediate layer positioned between the outer layer and the inner layer.

**Example Implementation 16:** The overwrap material of any preceding example implementation, or any combination of any preceding example implementations, wherein the outer layer comprises a paper material, the intermediate layer comprises a foil material, and the inner layer comprises an aerosol generating material.

**Example Implementation 17:** The overwrap material of any preceding example implementation, or any combination of any preceding example implementations, wherein the aerosol generating material comprises a cast sheet containing a fibrous material.

**Example Implementation 18:** The overwrap material of any preceding example implementation, or any combination of any preceding example implementations, wherein the aerosol generating material comprises a reconstituted tobacco sheet.

**Example Implementation 19:** The overwrap material of any preceding example implementation, or any combination of any preceding example implementations, wherein the overwrap material is configured to produce the initial inhalable substance in response to application of heat from an electrically generated heat source.

**Example Implementation 20:** The overwrap material of any preceding example implementation, or any combination of any preceding example implementations, wherein the overwrap material is configured to produce the initial inhalable substance in response to application of heat from a combustible carbon-based heat source.

These and other features, aspects, and advantages of the disclosure will be apparent from a reading of the following detailed description together with the accompanying drawings, which are briefly described below. The invention includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosed invention, in any of its various aspects and embodiments, should be viewed as intended to be combinable unless the context clearly dictates otherwise.

### BRIEF DESCRIPTION OF THE FIGURES

Having thus described aspects of the disclosure in the foregoing general terms, reference will now be made to the accompanying drawings, which are not necessarily drawn to scale, and wherein:
FIG. 1 illustrates a perspective view of an aerosol delivery device comprising a control body and an aerosol source member, wherein the aerosol source member and the control body are coupled to one another, according to an example implementation of the present disclosure;
FIG. 2 illustrates a perspective view of the aerosol delivery device of FIG. 1, wherein the aerosol source member and the control body are decoupled from one another, according to an example implementation of the present disclosure;
FIG. 3 illustrates a front schematic view of the aerosol delivery device of FIG. 1, wherein the aerosol source member and the control body are coupled to one another, according to an example implementation of the present disclosure;
FIG. 4 illustrates a cross-section schematic view of a portion of the substrate portion of the aerosol source member of FIG. 1, according to an example implementation of the present disclosure;
FIG. 5 illustrates a perspective view of an aerosol source member, according to an example implementation of the present disclosure; and
FIG. 6 illustrates a cross-section schematic view of a portion of the substrate portion of the aerosol source member of FIG. 5, according to an example implementation of the present disclosure.

### DETAILED DESCRIPTION

The present disclosure will now be described more fully hereinafter with reference to example implementations thereof. These example implementations are described so that this disclosure will be thorough and complete, and will fully convey the scope of the disclosure to those skilled in the art. Indeed, the disclosure may be embodied in many different forms and should not be construed as limited to the implementations set forth herein; rather, these implementations are provided so that this disclosure will satisfy applicable legal requirements. As used in the specification and the appended claims, the singular forms "a," "an," "the" and the like include plural referents unless the context clearly dictates otherwise. Also, while reference may be made herein to quantitative measures, values, geometric relationships or the like, unless otherwise stated, any one or more if not all of these may be absolute or approximate to account for acceptable variations that may occur, such as those due to engineering tolerances or the like.

As described hereinafter, example implementations of the present disclosure relate to aerosol generating substrates for use in an aerosol source members and aerosol source members for use with aerosol delivery devices. Some implementations of aerosol delivery devices according to the present disclosure use electrical energy to heat a material to form an inhalable substance (e.g., electrically heated tobacco products). Other implementations of aerosol delivery devices and/or aerosol source members according to the present disclosure use an ignitable heat source to heat a material (preferably without combusting the material to any significant degree) to form an inhalable substance (e.g., carbon heated tobacco products). Preferably, the material is heated without combusting the material to any significant degree. Components of such systems have the form of articles that are sufficiently compact to be considered hand-held devices. That is, use of components of preferred aerosol delivery devices does not result in the production of smoke in the sense that aerosol results principally from by-products of combustion or pyrolysis of tobacco, but rather, use of those preferred systems results in the production of vapors resulting from volatilization or vaporization of certain components incorporated therein. In some example implementations, components of aerosol delivery devices and/or aerosol source members may be characterized as electronic cigarettes, and those electronic cigarettes most preferably incorporate tobacco and/or components derived from tobacco, and hence deliver tobacco derived components in aerosol form.

Aerosol generating components of certain preferred aerosol delivery devices and/or aerosol source members may provide many of the sensations (e.g., inhalation and exhalation rituals, types of tastes or flavors, organoleptic effects, physical feel, use rituals, visual cues such as those provided by visible aerosol, and the like) of smoking a cigarette, cigar or pipe that is employed by lighting and burning tobacco (and hence inhaling tobacco smoke), without any substantial degree of combustion of any component thereof. For example, the user of an aerosol delivery device and/or an aerosol source member in accordance with some example implementations of the present disclosure can hold and use that component much like a smoker employs a traditional type of smoking article, draw on one end of that piece for inhalation of aerosol produced by that piece, take or draw puffs at selected intervals of time, and the like.

While the systems are generally described herein in terms of implementations associated with aerosol delivery devices and/or aerosol source members such as so-called "e-cigarettes" or "tobacco heating products," it should be understood that the mechanisms, components, features, and methods may be embodied in many different forms and associated with a variety of articles. For example, the description provided herein may be employed in conjunction with implementations of traditional smoking articles (e.g., cigarettes, cigars, pipes, etc.), heat-not-burn cigarettes, and related packaging for any of the products disclosed herein. Accordingly, it should be understood that the description of the mechanisms, components, features, and methods disclosed herein are discussed in terms of implementations relating to aerosol delivery devices by way of example only, and may be embodied and used in various other products and methods.

Aerosol delivery devices and/or aerosol source members of the present disclosure may also be characterized as being vapor-producing articles or medicament delivery articles. Thus, such articles or devices may be adapted so as to provide one or more substances (e.g., flavors and/or pharmaceutical active ingredients) in an inhalable form or state. For example, inhalable substances may be substantially in the form of a vapor (i.e., a substance that is in the gas phase at a temperature lower than its critical point). Alternatively, inhalable substances may be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like. The physical form of the inhalable substance is not necessarily limited by the nature of the inventive devices but rather may depend upon the nature of the medium and the inhalable substance itself as to whether it exists in a vapor state or an aerosol state. In some implementations, the terms "vapor" and "aerosol" may be interchangeable. Thus, for simplicity, the terms "vapor" and "aerosol" as used to describe aspects of the disclosure are understood to be interchangeable unless stated otherwise.

In some implementations, aerosol delivery devices of the present disclosure may comprise some combination of a power source (e.g., an electrical power source), at least one control component (e.g., means for actuating, controlling, regulating and ceasing power for heat generation, such as by controlling electrical current flow from the power source to other components of the article - e.g., a microprocessor, individually or as part of a microcontroller), a heating member (e.g., an electrical resistance heating element or other component and/or an inductive coil or other associated components and/or one or more radiant heating elements), and an aerosol source member that includes a substrate portion capable of yielding an aerosol upon application of sufficient heat. In some implementations the heating member may be located in the control body, and in other implementations the heating member may be located in the aerosol source member. In various implementations, a number of these components may be provided within an outer body or shell, which, in some implementations, may be referred to as a housing. The overall design of the outer body or shell may vary, and the format or configuration of the outer body that may define the overall size and shape of the aerosol delivery device may vary. Although other configurations are possible, in some implementations an elongated body resembling the shape of a cigarette or cigar may be a formed from a single, unitary housing or the elongated housing can be formed of two or more separable bodies. For example, an aerosol delivery device may comprise an elongated shell or body that may be substantially tubular in shape and, as such, resemble the shape of a conventional cigarette or cigar. In one example, all of the components of the aerosol delivery device are contained within one housing or body. In other implementations, an aerosol delivery device may comprise two or more housings that are joined and are separable. For example, an aerosol delivery device may possess at one end a control body comprising a housing containing one or more reusable components (e.g., an accumulator such as a rechargeable battery and/or rechargeable supercapacitor, and various electronics for controlling the operation of that article), and at the other end and removably coupleable thereto, an outer body or shell containing a disposable portion (e.g., a disposable flavor-containing aerosol source member).

In other implementations, aerosol source members of the present disclosure may generally include an ignitable heat source configured to heat a substrate material. The substrate material and/or at least a portion of the heat source may be covered in an outer wrap, or wrapping, a casing, a component, a module, a member, or the like. The overall design of the enclosure is variable, and the format or configuration of the enclosure that defines the overall size and shape of the aerosol source member is also variable. Although other configurations are possible, it may be desirable, in some aspects, that the overall design, size, and/or shape of these implementations resemble that of a conventional cigarette or cigar. In various aspects, the heat source may be capable of generating heat to aerosolize a substrate material that comprises, for example, a substrate material associated with an aerosol precursor composition, an extruded structure and/or substrate, tobacco and/or a tobacco related material, such as a material that is found naturally in tobacco that is isolated directly from the tobacco or synthetically prepared, in a solid or liquid form (e.g., beads, sheets, shreds, a wrap), or the like.

More specific formats, configurations and arrangements of various substrate materials, aerosol source members, and components within aerosol delivery devices of the present disclosure will be evident in light of the further disclosure provided hereinafter. Additionally, the selection of various aerosol delivery device components may be appreciated upon consideration of the commercially available electronic aerosol delivery devices. Further, the arrangement of the components within the aerosol delivery device may also be appreciated upon consideration of the commercially available electronic aerosol delivery devices.

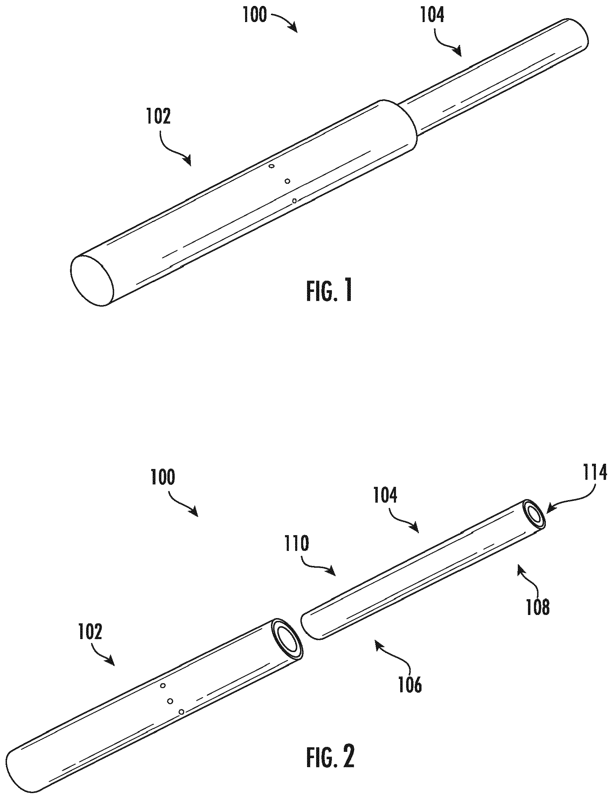

In this regard, FIG. 1 illustrates an aerosol delivery device **100** according to an example implementation of the present disclosure. The aerosol delivery device **100** may include a control body **102** and an aerosol source member **104.** In various implementations, the aerosol source member **104** and the control body **102** may be permanently or detachably aligned in a functioning relationship. In this regard, FIG. 1 illustrates the aerosol delivery device **100** in a coupled configuration, whereas FIG. 2 illustrates the aerosol delivery device **100** in a decoupled configuration. Various mechanisms may connect the aerosol source member **104** to the control body **102** to result in a threaded engagement, a press-fit engagement, an interference fit, a sliding fit, a magnetic engagement, or the like. Although an aerosol delivery device according to the present disclosure may take on a variety of implementations, as discussed in detail below, the use of the aerosol delivery device by a consumer will be similar in scope. The foregoing description of use of the aerosol delivery device is applicable to the various implementations described through minor modifications, which are apparent to the person of skill in the art in light of the further disclosure provided herein. The description of use, however, is not intended to limit the use of the articles of the present disclosure but is provided to comply with all necessary requirements of disclosure herein.

In various implementations, the aerosol delivery device **100** according to the present disclosure may have a variety of overall shapes, including, but not limited to an overall shape that may be defined as being substantially rod-like or substantially tubular shaped or substantially cylindrically shaped. In the implementations of FIGS. 1-2, the device **100** has a substantially round cross-section; however, other cross-sectional shapes (e.g., oval, square, triangle, variable, etc.) also are encompassed by the present disclosure. For example, in some implementations one or both of the control body **102** or the aerosol source member **104** (and/or any subcomponents) may have a substantially rectangular shape, such as a substantially rectangular cuboid shape (e.g., similar to a USB flash drive). In other implementations, one or both of the control body **102** or the aerosol source member **104** (and/or any subcomponents) may have other hand-held shapes. For example, in some implementations the control body **102** may have a small box shape, various pod mod shapes, or a fob-shape. Thus, such language that is descriptive of the physical shape of the article may also be applied to the individual components thereof, including the control body **102** and the aerosol source member **104.**

Alignment of the components within the aerosol delivery device of the present disclosure may vary across various implementations. In some implementations, the substrate portion may be positioned proximate a heating member so as to maximize aerosol delivery to the user. Other configurations, however, are not excluded. Generally, the heating member may be positioned sufficiently near the substrate portion so that heat from the heating member can volatilize the substrate portion (as well as, in some implementations, one or more flavorants, medicaments, or the like that may likewise be provided for delivery to a user) and form an aerosol for delivery to the user. When the heating member heats the substrate portion, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof, wherein such terms are also interchangeably used herein except where otherwise specified.

As noted above, the aerosol delivery device **100** of various implementations may incorporate a battery and/or other electrical power source to provide current flow sufficient to provide various functionalities to the aerosol delivery device, such as powering of the heating member, powering of control systems, powering of indicators, and the like. As will be discussed in more detail below, the power source may take on various implementations. Preferably, the power source may be able to deliver sufficient power to rapidly activate the heating member to provide for aerosol formation and power the aerosol delivery device through use for a desired duration of time. In some implementations, the power source is sized to fit conveniently within the aerosol delivery device so that the aerosol delivery device can be easily handled. Examples of useful power sources include lithium-ion batteries that are preferably rechargeable (e.g., a rechargeable lithium-manganese dioxide battery). In particular, lithium polymer batteries can be used as such batteries can provide increased safety. Other types of batteries - e.g., N50-AAA CADNICA nickel-cadmium cells - may also be used. Additionally, a preferred power source is of a sufficiently light weight to not detract from a desirable smoking experience. Some examples of possible power sources are described in U.S. Pat. No. 9,484,155 to Peckerar et al., and U.S. Pat. App. Pub. No. 2017/0112191 to Sur et al., filed October 21, 2015, the disclosures of which are incorporated herein by reference in their respective entireties.

In specific implementations, one or both of the control body **102** and the aerosol source member **104** may be referred to as being disposable or as being reusable. For example, the control body **102** may have a replaceable battery or a rechargeable battery, solid-state battery, thin-film solid-state battery, rechargeable supercapacitor or the like, and thus may be combined with any type of recharging technology, including connection to a wall charger, connection to a car charger (i.e., cigarette lighter receptacle), and connection to a computer, such as through a universal serial bus (USB) cable or connector (e.g., USB 2.0, 3.0, 3.1, USB Type-C), connection to a photovoltaic cell (sometimes referred to as a solar cell) or solar panel of solar cells, a wireless charger, such as a charger that uses inductive wireless charging (including for example, wireless charging according to the Qi wireless charging standard from the Wireless Power Consortium (WPC)), or a wireless radio frequency (RF) based charger. An example of an inductive wireless charging system is described in U.S. Pat. App. Pub. No. 2017/0112196 to Sur et al., which is incorporated herein by reference in its entirety. Further, in some implementations, the aerosol source member **104** may comprise a single-use device. A single use component for use with a control body is disclosed in U.S. Pat. No. 8,910,639 to Chang et al., which is incorporated herein by reference in its entirety.

In further implementations, the power source may also comprise a capacitor. Capacitors are capable of discharging more quickly than batteries and can be charged between puffs, allowing the battery to discharge into the capacitor at a lower rate than if it were used to power the heating member directly. For example, a supercapacitor - e.g., an electric double-layer capacitor (EDLC) - may be used separate from or in combination with a battery. When used alone, the supercapacitor may be recharged before each use of the article. Thus, the device may also include a charger component that can be attached to the smoking article between uses to replenish the supercapacitor.

Further components may be utilized in the aerosol delivery device of the present disclosure. For example, the aerosol delivery device may include a flow sensor that is sensitive either to pressure changes or air flow changes as the consumer draws on the article (e.g., a puff-actuated switch). Other possible current actuation/deactuation mechanisms may include a temperature actuated on/off switch or a lip pressure actuated switch. An example mechanism that can provide such puff-actuation capability includes a Model 163PC01D36 silicon sensor, manufactured by the MicroSwitch division of Honeywell, Inc., Freeport, Ill. With such a sensor, the heating member may be activated rapidly by a change in pressure when the consumer draws on the device. Other suitable current actuation/deactuation mechanisms may include a temperature actuated on/off switch or a lip pressure actuated switch. In addition, flow sensing devices, such as those using hot-wire anemometry principles, may be used to cause the energizing of a heating member sufficiently rapidly after sensing a change in air flow. A further puff actuated switch that may be used is a pressure differential switch, such as Model No. MPL-502-V, range A, from Micro Pneumatic Logic, Inc., Ft. Lauderdale, Fla. Another suitable puff actuated mechanism is a sensitive pressure transducer (e.g., equipped with an amplifier or gain stage) which is in turn coupled with a comparator for detecting a predetermined threshold pressure. Yet another suitable puff actuated mechanism is a vane which is deflected by airflow, the motion of which vane is detected by a movement sensing means. Yet another suitable actuation mechanism is a piezoelectric switch. Also useful is a suitably connected Honeywell MicroSwitch Microbridge Airflow Sensor, Part No. AWM 2100V from MicroSwitch Division of Honeywell, Inc., Freeport, Ill. Further examples of demand-operated electrical switches that may be employed in a heating circuit according to the present disclosure are described in U.S. Pat. No. 4,735,217 to Gerth et al., which is incorporated herein by reference in its entirety. Other suitable differential switches, analog pressure sensors, flow rate sensors, or the like, will be apparent to the skilled artisan with the knowledge of the present disclosure. In some implementations, a pressure-sensing tube or other passage providing fluid connection between the puff actuated switch and a receiving chamber may be included in the control body **102** so that pressure changes during draw are readily identified by the switch. Other examples of puff actuation devices that may be useful according to the present disclosure are disclosed in U.S. Pat. Nos. 4,922,901, 4,947,874, and 4,947,874, all to Brooks et al., U.S. Pat. No. 5,372,148 to McCafferty et al., U.S. Pat. No. 6,040,560 to Fleischhauer et al., and U.S. Pat. No. 7,040,314 to Nguyen et al., all of which are incorporated herein by reference in their entireties. In addition to, or as an alternate to those described above, manually activated switches may also be utilized.

In another example, an aerosol delivery device may comprise a first conductive surface configured to contact a first body part of a user holding the device, and a second conductive surface, conductively isolated from the first conductive surface, configured to contact a second body part of the user. As such, when the aerosol delivery device detects a change in conductivity between the first conductive surface and the second conductive surface, a vaporizer is activated to vaporize a substance so that the vapors may be inhaled by the user holding unit. The first body part and the second body part may be a lip or parts of a hand(s). The two conductive surfaces may also be used to charge a battery contained in the personal vaporizer unit. The two conductive surfaces may also form, or be part of, a connector that may be used to output data stored in a memory. Reference is made to U.S. Pat. No. 9,861,773 to Terry et al., which is incorporated herein by reference in its entirety.

In addition, U.S. Pat. No. 5,154,192 to Sprinkel et al. discloses indicators for smoking articles; U.S. Pat. No. 5,261,424 to Sprinkel, Jr. discloses piezoelectric sensors that can be associated with the mouth-end of a device to detect user lip activity associated with taking a draw and then trigger heating of a heating device; U.S. Pat. No. 5,372,148 to McCafferty et al. discloses a puff sensor for controlling energy flow into a heating load array in response to pressure drop through a mouthpiece; U.S. Pat. No. 5,967,148 to Harris et al. discloses receptacles in a smoking device that include an identifier that detects a non-uniformity in infrared transmissivity of an inserted component and a controller that executes a detection routine as the component is inserted into the receptacle; U.S. Pat. No. 6,040,560 to Fleischhauer et al. describes a defined executable power cycle with multiple differential phases; U.S. Pat. No. 5,934,289 to Watkins et al. discloses photonic-optronic components; U.S. Pat. No. 5,954,979 to Counts et al. discloses means for altering draw resistance through a smoking device; U.S. Pat. No. 6,803,545 to Blake et al. discloses specific battery configurations for use in smoking devices; U.S. Pat. No. 7,293,565 to Griffen et al. discloses various charging systems for use with smoking devices; U.S. Pat. No. 8,402,976 to Fernando et al. discloses computer interfacing means for smoking devices to facilitate charging and allow computer control of the device; U.S. Pat. No. 8,689,804 to Fernando et al. discloses identification systems for smoking devices; and PCT Pat. App. Pub. No. WO 2010/003480 by Flick discloses a fluid flow sensing system indicative of a puff in an aerosol generating system; all of the foregoing disclosures being incorporated herein by reference in their entireties.

Further examples of components related to electronic aerosol delivery articles and disclosing materials or components that may be used in the present device include U.S. Pat. No. 4,735,217 to Gerth et al.; U.S. Pat. No. 5,249,586 to Morgan et al.; U.S. Pat. No. 5,666,977 to Higgins et al.; U.S. Pat. No. 6,053,176 to Adams et al.; U.S. Pat. No. 6,164,287 to White; U.S. Pat No. 6,196,218 to Voges; U.S. Pat. No. 6,810,883 to Felter et al.; U.S. Pat. No. 6,854,461 to Nichols; U.S. Pat. No. 7,832,410 to Hon; U.S. Pat. No. 7,513,253 to Kobayashi; U.S. Pat. No. 7,896,006 to Hamano; U.S. Pat. No. 6,772,756 to Shayan; U.S. Pat. Nos. 8,156,944 and 8,375,957 to Hon; U.S. Pat. No. 8,794,231 to Thorens et al.; U.S. Pat. No. 8,851,083 to Oglesby et al.; U.S. Pat. Nos. 8,915,254 and 8,925,555 to Monsees et al.; U.S. Pat. No. 9,220,302 to DePiano et al.; U.S. Pat. App. Pub. Nos. 2006/0196518 and 2009/0188490 to Hon; U.S. Pat. App. Pub. No. 2010/0024834 to Oglesby et al.; U.S. Pat. App. Pub. No. 2010/0307518 to Wang; PCT Pat. App. Pub. No. WO 2010/091593 to Hon; and PCT Pat. App. Pub. No. WO 2013/089551 to Foo, each of which is incorporated herein by reference in its entirety. Further, U.S. Pat. App. Pub. No. 2017/0099877 to Worm et al., filed October 13, 2015, discloses capsules that may be included in aerosol delivery devices and fob-shape configurations for aerosol delivery devices, and is incorporated herein by reference in its entirety. A variety of the materials disclosed by the foregoing documents may be incorporated into the present devices in various implementations, and all of the foregoing disclosures are incorporated herein by reference in their entireties.

Referring to FIG. 2, in the depicted implementation, the aerosol source member **104** comprises a heated end **106,** which is configured to be inserted into the control body **102,** and a mouth end **108,** upon which a user draws to create the aerosol. At least a portion of the heated end **106** may include a substrate portion **110.** As will be discussed in more detail below, in various implementations, the aerosol source member **104,** or a portion thereof (e.g., the heated end **106** of the aerosol source member **102)** may be wrapped in an exterior overwrap material. In various implementations, the mouth end **108** of the aerosol source member **104** may include a filter **114,** which may, for example, be made of a cellulose acetate or polypropylene material. The filter **114** may additionally or alternatively contain strands of tobacco containing material, such as described in U.S. Pat. No. 5,025,814 to Raker et al., which is incorporated herein by reference in its entirety. In various implementations, the filter **114** may increase the structural integrity of the mouth end of the aerosol source member, and/or provide filtering capacity, if desired, and/or provide resistance to draw. In some implementations, the filter may comprise discrete segments. For example, some implementations may include a segment providing filtering, a segment providing draw resistance, a hollow segment providing a space for the aerosol to cool, a segment providing increased structural integrity, other filter segments, and any one or any combination of the above.

In various implementations, other components may exist between the substrate portion **110** and the mouth end **108** of the aerosol source member **104.** For example, in some implementations one or any combination of the following may be positioned between the substrate portion **110** and the mouth end **108** of the aerosol source member **104:** an air gap; a hollow tube structure; phase change materials for cooling air; flavor releasing media; ion exchange fibers capable of selective chemical adsorption; aerogel particles as filter medium; and other suitable materials. Some examples of possible phase change materials include, but are limited to, salts, such as AgNO₃, AlCl₃, TaCl₃, InCl₃, SnClz, AlI₃, and TiI₄; metals and metal alloys such as selenium, tin, indium, tin-zinc, indium-zinc, or indium-bismuth; and organic compounds such as D-mannitol, succinic acid, p-nitrobenzoic acid, hydroquinone and adipic acid. Other examples are described in U.S. Pat. No. 8,430,106 to Potter et al., which is incorporated herein by reference in its entirety.

As will be discussed in more detail below, the present disclosure is configured for use with a conductive and/or inductive heat source to heat a substrate material to form an aerosol. In various implementations, a conductive heat source may comprise a heating assembly that comprises a resistive heating member. Resistive heating members may be configured to produce heat when an electrical current is directed therethrough. Electrically conductive materials useful as resistive heating members may be those having low mass, low density, and moderate resistivity and that are thermally stable at the temperatures experienced during use. Useful heating members heat and cool rapidly, and thus provide for the efficient use of energy. Rapid heating of the member may be beneficial to provide almost immediate volatilization of an aerosol precursor material in proximity thereto. Rapid cooling prevents substantial volatilization (and hence waste) of the aerosol precursor material during periods when aerosol formation is not desired. Such heating members may also permit relatively precise control of the temperature range experienced by the aerosol precursor material, especially when time based current control is employed. Useful electrically conductive materials are preferably chemically non-reactive with the materials being heated (e.g., aerosol precursor materials and other inhalable substance materials) so as not to adversely affect the flavor or content of the aerosol or vapor that is produced. Some example, non-limiting, materials that may be used as the electrically conductive material include carbon, graphite, carbon/graphite composites, metals, ceramics such as metallic and non-metallic carbides, nitrides, oxides, silicides, inter-metallic compounds, cermets, metal alloys, and metal foils. In particular, refractory materials may be useful. Various, different materials can be mixed to achieve the desired properties of resistivity, mass, and thermal conductivity. In specific implementations, metals that can be utilized include, for example, nickel, chromium, alloys of nickel and chromium (e.g., nichrome), and steel. Materials that can be useful for providing resistive heating are described in U.S. Pat. No. 5,060,671 to Counts et al.; U.S. Pat. Nos. 5,093,894 to Deevi et al.; 5,224,498 to Deevi et al.; 5,228,460 to Sprinkel Jr., et al.; 5,322,075 to Deevi et al.; U.S. Pat. No. 5,353,813 to Deevi et al.; U.S. Pat. No. 5,468,936 to Deevi et al.; U.S. Pat. No. 5,498,850 to Das; U.S. Pat. No. 5,659,656 to Das; U.S. Pat. No. 5,498,855 to Deevi et al.; U.S. Pat. No. 5,530,225 to Hajaligol; U.S. Pat. No. 5,665,262 to Hajaligol; U.S. Pat. No. 5,573,692 to Das et al.; and U.S. Pat. No. 5,591,368 to Fleischhauer et al., the disclosures of which are incorporated herein by reference in their entireties.

In various implementations, a heating member may be provided in a variety of forms, such as in the form of a foil, a foam, a mesh, a hollow ball, a half ball, discs, spirals, fibers, wires, films, yarns, strips, ribbons, or cylinders. Such heating members often comprise a metal material and are configured to produce heat as a result of the electrical resistance associated with passing an electrical current therethrough. Such resistive heating members may be positioned in proximity to, and/or in direct contact with, the substrate portion. For example, in one implementation, a heating member may comprise a cylinder or other heating device located in the control body **102,** wherein the cylinder is constructed of one or more conductive materials, including, but not limited to, copper, aluminum, platinum, gold, silver, iron, steel, brass, bronze, carbon (e.g., graphite), or any combination thereof. In various implementations, the heating member may also be coated with any of these or other conductive materials. The heating member may be located proximate an engagement end of the control body **102,** and may be configured to substantially surround a portion of the heated end **106** of the aerosol source member **104** that includes the substrate portion **110.** In such a manner, the heating member may be located proximate the substrate portion **110** of the aerosol source member **104** when the aerosol source member is inserted into the control body **102.** In other examples, at least a portion of a heating member may penetrate at least a portion of an aerosol source member (such as, for example, one or more prongs and/or spikes that penetrate an aerosol source member), when the aerosol source member is inserted into the control body.

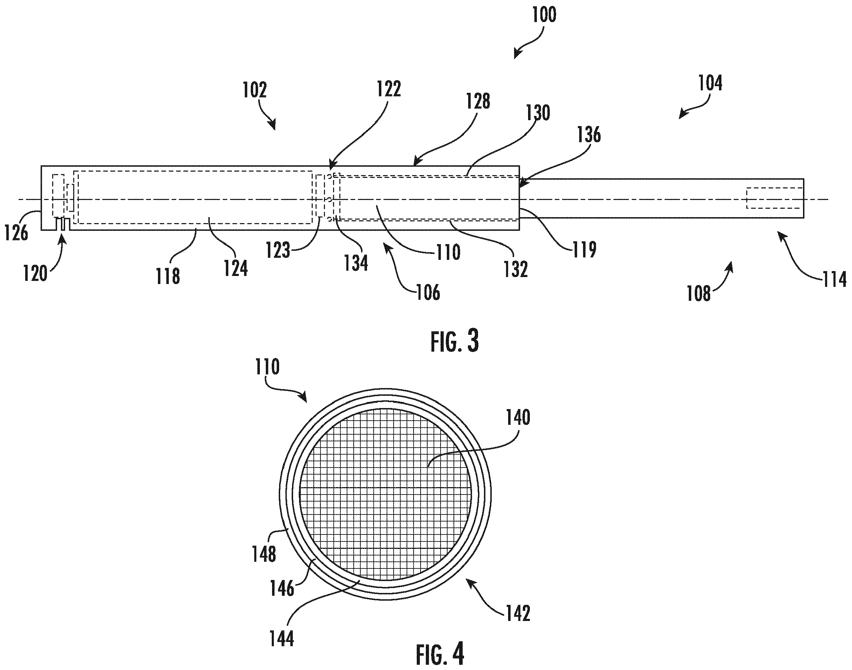

FIG. 3 illustrates a front schematic view of the aerosol delivery device of FIG. 1, wherein control body and the aerosol source member are coupled to one another, according to an example implementation of the present disclosure. As illustrated in the figures, the aerosol delivery device **100** of this example implementation includes a heating assembly **128** that includes a heating member **132,** in the form of a circumferential heater that substantially surrounds at least a portion of the heated end **106** of the aerosol source member **104.** In particular, the control body **102** of the depicted implementation comprises a housing **118** that includes an opening **119** defined in an engaging end thereof. The control body **102** also includes a flow sensor **120** (e.g., a puff sensor or pressure switch), a control component **123** (e.g., processing circuitry, individually or as part of a microcontroller, a printed circuit board (PCB) that includes a microprocessor and/or microcontroller, etc.), a power source **124** (e.g., a battery, which may be rechargeable, and/or a rechargeable supercapacitor), and an end cap that includes an indicator **126** (e.g., a light emitting diode (LED)). In one implementation, the indicator **126** may comprise one or more light emitting diodes, quantum dot-based light emitting diodes or the like. The indicator **126** may be in communication with the control component **123** and be illuminated, for example, when a user draws on the aerosol source member **104,** when coupled to the control body **102,** as detected by the flow sensor **120.**

Other indices of operation are also encompassed by the present disclosure. For example, visual indicators of operation also include changes in light color or intensity to show progression of the smoking experience. Tactile indicators of operation and sound indicators of operation similarly are encompassed by the present disclosure. Moreover, combinations of such indicators of operation also are suitable to be used in a single smoking article. According to another aspect, the device may include one or more indicators or indicia, such as, for example, a display configured to provide information corresponding to the operation of the smoking article such as, for example, the amount of power remaining in the power source, progression of the smoking experience, indication corresponding to activating a heat source, and/or the like.

Examples of possible power sources are described in U.S. Pat. No. 9,484,155 to Peckerar et al., and U.S. Pat. App. Pub. No. 2017/0112191 to Sur et al., filed October 21, 2015, the disclosures of which are incorporated herein by reference in their respective entireties. With respect to the flow sensor, representative current regulating components and other current controlling components including various microcontrollers, sensors, and switches for aerosol delivery devices are described in U.S. Pat. No. 4,735,217 to Gerth et al., U.S. Pat. Nos. 4,922,901, 4,947,874, and 4,947,875, all to Brooks et al., U.S. Pat. No. 5,372,148 to McCafferty et al., U.S. Pat. No. 6,040,560 to Fleischhauer et al., U.S. Pat. No. 7,040,314 to Nguyen et al., and U.S. Pat. No. 8,205,622 to Pan, all of which are incorporated herein by reference in their entireties. Reference also is made to the control schemes described in U.S. Pat. No. 9,423,152 to Ampolini et al., which is incorporated herein by reference in its entirety.

Still further components may be utilized in the aerosol delivery device of the present disclosure. For example, U.S. Pat. No. 5,154,192 to Sprinkel et al. discloses indicators for smoking articles; U.S. Pat. No. 5,261,424 to Sprinkel, Jr. discloses piezoelectric sensors that can be associated with the mouth-end of a device to detect user lip activity associated with taking a draw and then trigger heating of a heating device; U.S. Pat. No. 5,372,148 to McCafferty et al. discloses a puff sensor for controlling energy flow into a heating load array in response to pressure drop through a mouthpiece; U.S. Pat. No. 5,967,148 to Harris et al. discloses receptacles in a smoking device that include an identifier that detects a non-uniformity in infrared transmissivity of an inserted component and a controller that executes a detection routine as the component is inserted into the receptacle; U.S. Pat. No. 6,040,560 to Fleischhauer et al. describes a defined executable power cycle with multiple differential phases; U.S. Pat. No. 5,934,289 to Watkins et al. discloses photonic-optronic components; U.S. Pat. No. 5,954,979 to Counts et al. discloses means for altering draw resistance through a smoking device; U.S. Pat. No. 6,803,545 to Blake et al. discloses specific battery configurations for use in smoking devices; U.S. Pat. No. 7,293,565 to Griffen et al. discloses various charging systems for use with smoking devices; U.S. Pat. No. 8,402,976 to Fernando et al. discloses computer interfacing means for smoking devices to facilitate charging and allow computer control of the device; U.S. Pat. No. 8,689,804 to Fernando et al. discloses identification systems for smoking devices; and PCT Pat. App. Pub. No. WO 2010/003480 by Flick discloses a fluid flow sensing system indicative of a puff in an aerosol generating system; all of the foregoing disclosures being incorporated herein by reference in their entireties.

Further examples of components related to electronic aerosol delivery articles and disclosing materials or components that may be used in the present article include U.S. Pat. No. 4,735,217 to Gerth et al.; U.S. Pat. No. 5,249,586 to Morgan et al.; U.S. Pat. No. 5,666,977 to Higgins et al.; U.S. Pat. No. 6,053,176 to Adams et al.; U.S. Pat. No. 6,164,287 to White; U.S. Pat No. 6,196,218 to Voges; U.S. Pat. No. 6,810,883 to Felter et al.; U.S. Pat. No. 6,854,461 to Nichols; U.S. Pat. No. 7,832,410 to Hon; U.S. Pat. No. 7,513,253 to Kobayashi; U.S. Pat. No. 7,896,006 to Hamano; U.S. Pat. No. 6,772,756 to Shayan; U.S. Pat. Nos. 8,156,944 and 8,375,957 to Hon; U.S. Pat. No. 8,794,231 to Thorens et al.; U.S. Pat. No. 8,851,083 to Oglesby et al.; U.S. Pat. Nos. 8,915,254 and 8,925,555 to Monsees et al.; U.S. Pat. No. 9,220,302 to DePiano et al.; U.S. Pat. App. Pub. Nos. 2006/0196518 and 2009/0188490 to Hon; U.S. Pat. App. Pub. No. 2010/0024834 to Oglesby et al.; U.S. Pat. App. Pub. No. 2010/0307518 to Wang; PCT Pat. App. Pub. No. WO 2010/091593 to Hon; and PCT Pat. App. Pub. No. WO 2013/089551 to Foo, each of which is incorporated herein by reference in its entirety. Further, U.S. Pat. App. Pub. No. U.S. Pat. App. Pub. No. 2017-0099877 to Worm et al., filed October 13, 2015, discloses capsules that may be included in aerosol delivery devices and fob-shape configurations for aerosol delivery devices, and is incorporated herein by reference in its entirety. A variety of the materials disclosed by the foregoing documents may be incorporated into the present devices in various implementations, and all of the foregoing disclosures are incorporated herein by reference in their entireties.

Referring back to FIG. 3, the control body **102** of the depicted implementation includes a heating assembly **128** configured to heat the substrate portion **110** of the aerosol source member **104.** Although the heating assembly of various implementations of the present disclosure may take a variety of forms, in the particular implementation depicted in FIG. 3, the heating assembly **128** comprises an outer cylinder **130** that includes a circumferential heating member **132.** Examples of circumferential heating members are described in U.S. Pat. App. No. 15/916,834, to Sur et al., which is incorporated herein by reference in its entirety.

As illustrated, the heating assembly **128** may extend proximate an engagement end of the housing **118,** and may be configured to substantially surround a portion of the heated end **106** of the aerosol source member **104** that includes the inhalable substance medium **110.** In such a manner, the heating assembly **128** may define a generally tubular configuration. As illustrated in FIG. 3, the heating cylinder **130** creates a receiving chamber **136.** A receiving base **134** is disposed at the end of the receiving chamber **136** opposite the opening **119.** As such, the heating cylinder **130** may also serve to facilitate proper positioning of the aerosol source member **104** when the aerosol source member **104** is inserted into the housing **118.** In various implementations, the heating cylinder **130** of the heating assembly **128** may engage an internal surface of the housing **118** to provide for alignment of the heating assembly **128** with respect to the housing **118.** Thereby, as a result of the fixed coupling between the heating assembly **128,** a longitudinal axis of the heating assembly **128** may extend substantially parallel to a longitudinal axis of the housing **118.** In particular, the heating cylinder **130** may extend from the opening **119** of the housing **118** to receiving base **134** to create the receiving chamber **136.** In the illustrated implementation, the heating cylinder **130** is configured to guide the aerosol source member **104** into the proper position (e.g., lateral position) with respect to the control body **102.**

In various implementations, the control body **102** is configured such that when the aerosol source member **104** is inserted into the control body **102,** the heating cylinder **130** is proximate at least a portion of the substrate portion **110** of the heated end **106** of the aerosol source member **104.** In the depicted implementation, the control body **102** is configured such that when the aerosol source member **104** is inserted into the control body **102,** the heating cylinder **130** is in direct contact with an outer surface of the aerosol source member **104,** and proximate the heated end **106** of the aerosol source member **104.**

During use, the consumer initiates heating of the heating assembly **128,** and in particular, the heating member **132.** Due to the spatial relationship of the heating member **132** and the heating cylinder **130,** by heat transfer this also initiates heating of the substrate portion **110** (or a specific layer thereof). As will be discussed in more detail below, heating of the substrate portion **110** releases one or more inhalable substances from the aerosol source member **104.** When the consumer inhales on the mouth end **108** of the aerosol source member **104,** air is drawn into the aerosol source member **104** through openings or apertures **122** in the control body **102.** The combination of the drawn air and the released inhalable substance is inhaled by the consumer as the drawn materials exit the mouth end **108** of the aerosol source member **104.** In some implementations, to initiate heating, the consumer may manually actuate a pushbutton or similar component that causes the heating member of the heating assembly to receive electrical energy from the battery or other energy source. For example, in some implementations the consumer may use the pushbutton to energize the heating member **132.** Similar functionality tied to the pushbutton may be achieved by other mechanical means or non-mechanical means (e.g., magnetic or electromagnetic). Thusly, activation of the heating member **132** may be controlled by a single pushbutton. Alternatively, multiple pushbuttons may be provided to control various actions separately. One or more pushbuttons present may be substantially flush with the casing of the control body **102.** The electrical energy may be supplied for a pre-determined length of time or may be manually controlled. In some implementations, flow of electrical energy does not substantially proceed in between puffs on the device (although energy flow may proceed to maintain a baseline temperature greater than ambient temperature - e.g., a temperature that facilitates rapid heating to the active heating temperature). In the depicted implementation, however, heating is initiated by the puffing action of the consumer through use of one or more sensors, such as flow sensor **120.** Once the puff is discontinued, heating will stop or be reduced. When the consumer has taken a sufficient number of puffs so as to have released a sufficient amount of the inhalable substance (e.g., an amount sufficient to equate to a typical smoking experience), the aerosol source member **104** may be removed from the control body **102** and discarded. In some implementations, further sensing elements, such as capacitive sensing elements and other sensors, may be used as discussed in U.S. Pat. App. No. 15/707,461 to Phillips et al., which is incorporated herein by reference in its entirety.

Referring back to FIG. 3, the heated end **106** of the aerosol source member **104** is sized and shaped for insertion into the control body **102.** In various implementations, the receiving chamber **136** of the control body **102** may be characterized as being defined by a wall with an inner surface and an outer surface, the inner surface defining the interior volume of the receiving chamber **136.** For example, in the depicted implementations, the heating cylinder **130** defines an inner surface defining the interior volume of the receiving chamber **136.** Thus, the largest outer diameter (or other dimension depending upon the specific cross-sectional shape of the implementations) of the aerosol source member **104** may be sized to be less than the inner diameter (or other dimension) at the inner surface of the wall of the open end of the receiving chamber **136** in the control body **102.** In some implementations, the difference in the respective diameters may be sufficiently small so that the aerosol source member fits snugly into the receiving chamber **136,** and frictional forces prevent the aerosol source member **104** from being moved without an applied force. On the other hand, the difference may be sufficient to allow the aerosol source member **104** to slide into or out of the receiving chamber **136** without requiring undue force.

In some implementations, the overall size of the aerosol delivery device **100** may take on a size that is comparative to a cigarette or cigar shape. Thus, the device may have a diameter of about 5 mm to about 25 mm, about 5 mm to about 20 mm, about 6 mm to about 15 mm, or about 6 mm to about 10 mm. In various implementations, such dimension may particularly correspond to the outer diameter of the control body **102.** In some implementations, the aerosol source member **104** may have a diameter of between about 4mm and about 6mm. In addition, the control body **102** and the aerosol source member may likewise be characterized in relation to overall length. For example, in some implementations the control body may have a length of about 40 mm to about 120 mm, about 45 mm to about 110 mm, or about 50 mm to about 100 mm. The aerosol source member may have a length of about 20 mm to about 60 mm, about 25 mm to about 55 mm, or about 30 mm to about 50 mm.

As noted, the control body **102** of the depicted implementation includes a control component **123** that controls the various functions of the aerosol delivery device **100,** including providing power to the heating member **132.** For example, the control component **123** may include a control circuit (which may be connected to further components, as further described herein) that is connected by electrically conductive wires (not shown) to the power source **124.** In various implementations, the control circuit may control when and how the heating assembly **128,** and particularly the heating member **132,** receives electrical energy to heat the heating cylinder **130** and thus the substrate portion **110** for release of the inhalable substance for inhalation by a consumer. In some implementations, such control may be activated by a flow sensor and/or actuation of pressure sensitive switches or the like, which are described in greater detail hereinafter.

As noted, the control components may be configured to closely control the amount of heat provided to substrate portion **110.** While the heat needed to volatilize the aerosol-forming substance in a sufficient volume to provide a desired dosing of the inhalable substance for a single puff can vary for each particular substance used, in some implementations one or more of the heating circuits of the heating member may heat to a temperature of at least 120 °C, at least 130 °C, or at least 140 °C. In some implementations, in order to volatilize an appropriate amount of the aerosol-forming substance and thus provide a desired amount of the inhalable substance, the heating temperature may be at least 150 °C, at least 200 °C, at least 220 °C, at least 300 °C, or at least 350 °C. It can be particularly desirable, however, to avoid heating to temperatures substantially in excess of about 550 °C in order to avoid degradation and/or excessive, premature volatilization of the aerosol-forming substance. Heating specifically should be at a sufficiently low temperature and sufficiently short time so as to avoid significant combustion (preferably any combustion) of the inhalable substance medium. The present disclosure may particularly provide the components of the present device in combinations and modes of use that will yield the inhalable substance in desired amounts at relatively low temperatures. As such, yielding may refer to one or both of generation of the aerosol within the device and delivery out of the device to a consumer. In specific implementations, the heating temperature may be about 130 °C to about 310 °C, about 140 °C to about 300 °C, about 150 °C to about 290 °C, about 170 °C to about 270 °C, or about 180 °C to about 260 °C. In other implementations, the heating temperature may be about 210 °C to about 390 °C, about 220 °C to about 380 °C, about 230 °C to about 370 °C, about 250 °C to about 350 °C, or about 280 °C to about 320 °C.

The duration of heating may be controlled by a number of factors, as discussed in greater detail hereinbelow. Heating temperature and duration may depend upon the desired volume of aerosol and ambient air that is desired to be drawn through aerosol delivery device, as further described herein. The duration, however, may be varied depending upon the heating rate of the heating member, as the device may be configured such that the heating member is energized only until a desired temperature is reached. Alternatively, the duration of heating may be coupled to the duration of a puff on the article by a consumer. Generally, the temperature and time of heating will be controlled by one or more components contained in the control housing, as noted above.

In various implementations, the electrical heating assembly may include any device suitable to provide heat sufficient to facilitate release of the inhalable substance for inhalation by a consumer. In certain implementations, the electrical heating assembly may include a resistance heating member. Useful heating members may be those having low mass, low density, and moderate resistivity and that are thermally stable at the temperatures experienced during use. Useful heating members heat and cool rapidly, and thus provide for the efficient use of energy. Rapid heating of the element also provides almost immediate volatilization of the aerosol-forming substance. Rapid cooling prevents substantial volatilization (and hence waste) of the aerosol-forming substance during periods when aerosol formation is not desired. Such heating members also permit relatively precise control of the temperature range experienced by the aerosol-forming substance, especially when time-based current control is employed. Useful heating members also are chemically non-reactive with the materials comprising the inhalable substance medium being heated so as not to adversely affect the flavor or content of the aerosol or vapor that is produced. Some example, non-limiting, materials that may comprise the heating member include carbon, graphite, carbon/graphite composites, metals, metallic and non-metallic carbides, nitrides, silicides, inter-metallic compounds, cermets, metal alloys, and metal foils. In particular, refractory materials may be useful. Various, different materials can be mixed to achieve the desired properties of resistivity, mass, thermal conductivity, and surface properties. In some implementations, refractory materials may be useful. Various, different materials may be mixed to achieve the desired properties of resistivity, mass, and thermal conductivity. In specific aspects, metals that are able to be utilized include, for example, nickel, chromium, alloys of nickel and chromium (e.g., nichrome), and steel. Materials that may be useful for providing resistance or resistive heating are described in U.S. Pat. No. 5,060,671 to Counts et al.; U.S. Pat. No. 5,093,894 to Deevi et al.; 5,224,498 to Deevi et al.; U.S. Pat. No. 5,228,460 to Sprinkel Jr., et al.; U.S. Pat. No. 5,322,075 to Deevi et al.; U.S. Pat. No. 5,353,813 to Deevi et al.; U.S. Pat. No. 5,468,936 to Deevi et al.; U.S. Pat. No. 5,498,850 to Das; U.S. Pat. No. 5,659,656 to Das; U.S. Pat. No. 5,498,855 to Deevi et al.; U.S. Pat. No. 5,530,225 to Hajaligol; U.S. Pat. No. 5,665,262 to Hajaligol; U.S. Pat. No. 5,573,692 to Das et al.; and U.S. Pat. No. 5,591,368 to Fleischhauer et al., the disclosures of which are incorporated herein by reference in their entireties.

As noted, in various implementations the control body **102** may include one or more openings or apertures **122** therein for allowing entrance of ambient air into the interior of the receiving chamber **136.** In such a manner, in some implementations the receiving base **134** may also include apertures. Thus, in some implementations when a consumer draws on the mouth end of the aerosol source member **104,** air can be drawn through the apertures of the control body **102** and the receiving base **134** into the receiving chamber **136,** pass into the aerosol source member **104,** and be drawn through the inhalable substance medium **110** of the aerosol source member **104** for inhalation by the consumer. In some implementations, the drawn air carries the inhalable substance through the optional filter **114** and out of an opening at the mouth end **108** of the aerosol source member **104.** With the heating member **132** positioned inside the inhalable substance medium **110,** the heating member **132** may be activated to heat the inhalable substance medium **110** and cause release of the inhalable substance through the aerosol source member **104.**

In some implementations, it may be useful to provide some indication of when the aerosol source member **104** has achieved the proper distance of insertion into the receiving chamber **136** such that the heating member **132** is positioned in the inhalable substance medium **110.** For example, the aerosol source member **104** may include one or more markings on the exterior thereof (e.g., on the outer surface of the aerosol source member **104).** In other implementations, a single mark may indicate the depth of insertion required to achieve this position. Alternatively, proper insertion distance may be indicated by the aerosol source member **104** "bottoming out" against the base of the receiving chamber **136,** such as, for example, against the receiving base **134,** or any other such means that may enable a consumer to recognize and understand that the aerosol source member **104** has been inserted sufficiently in the receiving chamber **136** to position the heating member **132** in the proper location relative to the inhalable substance medium **110.**

When the consumer draws on the mouth end of the device **100,** the current actuation means may permit unrestricted or uninterrupted flow of current through the resistance heating member **132** to generate heat rapidly. Because of the rapid heating, it can be useful to include current regulating components to (i) regulate current flow through the heating member to control heating of the resistance element and the temperature experienced thereby, and (ii) prevent overheating and degradation of the substrate portion **110.** In some implementations, the current regulating circuit may be time-based. Specifically, such a circuit may include a means for permitting uninterrupted current flow through the heating member for an initial time period during draw, and a timer means for subsequently regulating current flow until draw is completed. For example, the subsequent regulation can include the rapid on-off switching of current flow (e.g., on the order of about every 1 to 50 milliseconds) to maintain the heating member within the desired temperature range. Further, regulation may comprise simply allowing uninterrupted current flow until the desired temperature is achieved then turning off the current flow completely. The heating member may be reactivated by the consumer initiating another puff on the article (or manually actuating the pushbutton, depending upon the specific switch implementation employed for activating the heater). Alternatively, the subsequent regulation can involve the modulation of current flow through the heating member to maintain the heating member within a desired temperature range. In some implementations, so as to release the desired dosing of the inhalable substance, the heating member may be energized for a duration of about 0.2 second to about 5.0 seconds, about 0.3 second to about 4.0 seconds, about 0.4 second to about 3.0 seconds, about 0.5 second to about 2.0 seconds, or about 0.6 second to about 1.5 seconds. One example of a time-based current regulating circuit can include a transistor, a timer, a comparator, and a capacitor. Suitable transistors, timers, comparators, and capacitors are commercially available and will be apparent to the skilled artisan. Example timers are those available from NEC Electronics as C-1555C and from General Electric Intersil, Inc. as ICM7555, as well as various other sizes and configurations of so-called "555 Timers". An example of a comparator is available from National Semiconductor as LM311. Further description of such time-based current regulating circuits is provided in U.S. Pat. No. 4,947,874 to Brooks et al., which is incorporated herein by reference in its entirety.

In light of the foregoing, it can be seen that a variety of mechanisms can be employed to facilitate actuation/deactuation of current to the heating member. For example, the device may include a timer for regulating current flow in the article (such as during draw by a consumer). The device may further include a timer responsive switch that enables and disables current flow to the heating member. Current flow regulation also can comprise use of a capacitor and components for charging and discharging the capacitor at a defined rate (e.g., a rate that approximates a rate at which the heating member heats and cools). Current flow specifically may be regulated such that there is uninterrupted current flow through the heating member for an initial time period during draw, but the current flow may be turned off or cycled alternately off and on after the initial time period until draw is completed. Such cycling may be controlled by a timer, as discussed above, which can generate a preset switching cycle. In specific implementations, the timer may generate a periodic digital wave form. The flow during the initial time period further may be regulated by use of a comparator that compares a first voltage at a first input to a threshold voltage at a threshold input and generates an output signal when the first voltage is equal to the threshold voltage, which enables the timer. Such implementations further can include components for generating the threshold voltage at the threshold input and components for generating the threshold voltage at the first input upon passage of the initial time period. In some implementations, a resistance temperature detector (RTD) may be used to control, at least partially, puff duration and temperature.

As noted above, the power source **124** used to provide power to the various electrical components of the device **100** may take on various implementations. Preferably, the power source is able to deliver sufficient energy to rapidly heat the heating member in the manner described above and power the device through use with multiple aerosol source members **104** while still fitting conveniently in the device **100.** One example of a power source is a TKI-1550 rechargeable lithium-ion battery produced by Tadiran Batteries GmbH of Germany. In another implementation, a useful power source may be a N50-AAA CADNICA nickel-cadmium cell produced by Sanyo Electric Company, Ltd., of Japan. In other implementations, a plurality of such batteries, for example providing 1.2-volts each, may be connected in series. Other power sources, such as rechargeable lithium-manganese dioxide batteries, may also be used. Any of these batteries or combinations thereof may be used in the power source, but rechargeable batteries are preferred because of cost and disposal considerations associated with disposable batteries. In implementations where rechargeable batteries are used, the power source **124** may further include charging contacts for interaction with corresponding contacts in a conventional recharging unit (not shown) deriving power from a standard 120-volt AC wall outlet, or other sources such as an automobile electrical system or a separate portable power supply. In further implementations, the power source may also comprise a capacitor. Capacitors are capable of discharging more quickly than batteries and can be charged between puffs, allowing the battery to discharge into the capacitor at a lower rate than if it were used to power the heating member directly. For example, a supercapacitor - i.e., an electric double-layer capacitor (EDLC) - may be used separate from or in combination with a battery. When used alone, the supercapacitor may be recharged before each use of the device **100.** Thus, the present disclosure also may include a charger component that can be attached to the device between uses to replenish the supercapacitor. Thin film batteries may be used in certain implementations of the present disclosure.

FIG. 4 illustrates a cross-section schematic view of a portion of the aerosol source member of FIG. 1, according to an example implementation of the present disclosure. In particular, FIG. 4 illustrates a portion of the substrate portion **110** of the aerosol source member **104,** which comprises a substrate material **140** substantially surrounded by an overwrap material **142.** In various implementations the substrate material is configured to generate an inhalable substance upon the application of heat thereupon, and may, or may not, include tobacco derived materials. In one implementation, the substrate material may comprise a blend of flavorful and aromatic tobaccos in cut filler form. In another implementation, the substrate material may comprise a reconstituted tobacco material, such as described in U.S. Pat. No. 4,807,809 to Pryor et al.; U.S. Pat. No. 4,889,143 to Pryor et al. and U.S. Pat. No. 5,025,814 to Raker, the disclosures of which are incorporated herein by reference in their entirety. Additionally, a reconstituted tobacco material may include a reconstituted tobacco paper for the type of cigarettes described in Chemical and Biological Studies on New Cigarette Prototypes that Heat Instead of Burn Tobacco, R. J. Reynolds Tobacco Company Monograph (1988), the contents of which are incorporated herein by reference in its entirety. For example, a reconstituted tobacco material may include a sheet-like material containing tobacco and/or tobacco-related materials. As such, in some implementations, the substrate material may be formed from a wound roll of a reconstituted tobacco material. In another implementation, the substrate material may be formed from shreds, strips, and/or the like of a reconstituted tobacco material. In another implementation, the tobacco sheet may comprise overlapping layers (e.g., a gathered web), which may, or may not, include heat conducting constituents. Examples of substrate portions that include a series of overlapping layers (e.g., gathered webs) of an initial substrate sheet formed by the fibrous filler material, aerosol forming material, and plurality of heat conducting constituents are described in U.S. Pat. App. No. 15/905,320, filed on February 26, 2018, and titled *Heat Conducting Substrate for Electrically Heated Aerosol Delivery Device,* which is incorporated herein by reference in its entirety.

In some implementations, the substrate material may include a plurality of microcapsules, beads, granules, and/or the like having a tobacco-related material. For example, a representative microcapsule may be generally spherical in shape, and may have an outer cover or shell that contains a liquid center region of a tobacco-derived extract and/or the like. In some implementations, the substrate material may include a plurality of microcapsules each formed into a hollow cylindrical shape. In some implementations, the substrate material may include a binder material configured to maintain the structural shape and/or integrity of the plurality of microcapsules formed into the hollow cylindrical shape. In some implementations, the substrate material may comprise a porous monolith that may, or may not, include tobacco materials. Some non-tobacco implementations may include, for example, one or more flavorants.

Tobacco employed in the substrate material can vary and may include, for example, flue-cured tobacco, burley tobacco, Oriental tobacco or Maryland tobacco, dark tobacco, dark-fired tobacco and *Rustica* tobaccos, as well as other rare or specialty tobaccos, or blends thereof. Tobacco materials also can include so-called "blended" forms and processed forms, such as processed tobacco stems (e.g., cut-rolled or cut-puffed stems), volume expanded tobacco (e.g., puffed tobacco, such as dry ice expanded tobacco (DIET), preferably in cut filler form), and/or reconstituted tobaccos (e.g., reconstituted tobaccos manufactured using paper-making type or cast sheet type processes). Various representative tobacco types, processed types of tobaccos, and types of tobacco blends are set forth in U.S. Pat. Nos. 4,836,224 to Lawson et al.; 4,924,888 to Perfetti et al.; 5,056,537 to Brown et al.; 5,159,942 to Brinkley et al.; 5,220,930 to Gentry; 5,360,023 to Blakley et al.; 6,701,936 to Shafer et al.; 7,011,096 to Li et al.; and 7,017,585 to Li et al.; 7,025,066 to Lawson et al.; U.S. Pat. App. Pub. No. 2004-0255965 to Perfetti et al.; PCT Pat. App. Pub. No. WO 02/37990 to Bereman; and Bombick et al., Fund. Appl. Toxicol., 39, p. 11-17 (1997); which are incorporated herein by reference in their entireties. Further examples of tobacco compositions that may be useful are disclosed in U.S. Pat. No. 7,726,320 to Robinson et al., which is incorporated herein by reference in its entirety. In some implementations, the milled tobacco material may comprise a blend of flavorful and aromatic tobaccos. In another implementation, the tobacco material may comprise a reconstituted tobacco material, such as described in U.S. Pat. No. 4,807,809 to Pryor et al.; U.S. Pat. No. 4,889,143 to Pryor et al. and U.S. Pat. No. 5,025,814 to Raker, the disclosures of which are incorporated herein by reference in their entirety. Additionally, a reconstituted tobacco material may include a reconstituted tobacco paper for the type of cigarettes described in Chemical and Biological Studies on New Cigarette Prototypes that Heat Instead of Burn Tobacco, R. J. Reynolds Tobacco Company Monograph (1988), the contents of which are incorporated herein by reference in its entirety.

In still other implementations of the present disclosure, the substrate material may include an extruded structure that includes, or is essentially comprised of a tobacco, a tobacco related material, glycerin, water, inert materials (such as calcium carbonate), and/or a binder material, although certain formulations may exclude the binder material. In various implementations, suitable binder materials may include alginates, such as ammonium alginate, propylene glycol alginate, potassium alginate, and sodium alginate. Alginates, and particularly high viscosity alginates, may be employed in conjunction with controlled levels of free calcium ions. Other suitable binder materials include hydroxypropylcellulose such as Klucel H from Aqualon Co.; hydroxypropylmethylcellulose such as Methocel K4MS from The Dow Chemical Co.; hydroxyethylcellulose such as Natrosol 250 MRCS from Aqualon Co.; microcrystalline cellulose such as Avicel from FMC; methylcellulose such as Methocel A4M from The Dow Chemical Co.; and sodium carboxymethyl cellulose such as CMC 7HF and CMC 7H4F from Hercules Inc. Still other possible binder materials include starches (e.g., corn starch), guar gum, carrageenan, locust bean gum, pectins and xanthan gum. In some implementations, combinations or blends of two or more binder materials may be employed. Other examples of binder materials are described, for example, in U.S. Pat. No. 5,101,839 to Jakob et al.; and U.S. Pat. No. 4,924,887 to Raker et al., each of which is incorporated herein by reference in its entirety. In some implementations, the aerosol forming material may be provided as a portion of the binder material (e.g., propylene glycol alginate). In addition, in some implementations, the binder material may comprise nanocellulose derived from a tobacco or other biomass.

In some implementations, the substrate material may include an extruded material, as described in U.S. Pat. App. Pub. No. 2012/0042885 to Stone et al., which is incorporated herein by reference in its entirety. In yet another implementation, the substrate material may include an extruded structure and/or substrate formed from marumarized and/or non-marumarized tobacco. Marumarized tobacco is known, for example, from U.S. Pat. No. 5,105,831 to Banerjee, et al., which is incorporated by reference herein in its entirety. Marumarized tobacco includes about 20 to about 50 percent (by weight) tobacco blend in powder form, with glycerol (at about 20 to about 30 percent weight), calcium carbonate (generally at about 10 to about 60 percent by weight, often at about 40 to about 60 percent by weight), along with binder agents, as described herein, and/or flavoring agents. In various implementations, the extruded material may have one or more longitudinal openings.

In various implementations, the substrate material may take on a variety of conformations based upon the various amounts of materials utilized therein. For example, a sample substrate material may comprise up to approximately 98% by weight, up to approximately 95% by weight, or up to approximately 90% by weight of a tobacco and/or tobacco related material. A sample substrate material may also comprise up to approximately 25% by weight, approximately 20% by weight, or approximately 15% by weight water - particularly approximately 2% to approximately 25%, approximately 5% to approximately 20%, or approximately 7% to approximately 15% by weight water. Flavors and the like (which include, for example, medicaments, such as nicotine) may comprise up to approximately 10%, up to about 8%, or up to about 5% by weight of the aerosol delivery component.

Additionally or alternatively, the substrate material may include an extruded structure and/or a substrate that includes or essentially is comprised of tobacco, glycerin, water, and/or binder material, and is further configured to substantially maintain its structure throughout the aerosol-generating process. That is, the substrate material may be configured to substantially maintain its shape (e.g., the substrate material does not continually deform under an applied shear stress) throughout the aerosol-generating process. Although such an example substrate material may include liquids and/or some moisture content, the substrate may remain substantially solid throughout the aerosol-generating process and may substantially maintain structural integrity throughout the aerosol-generating process. Example tobacco and/or tobacco related materials suitable for a substantially solid substrate material are described in U.S. Pat. App. Pub. No. 2015/0157052 to Ademe et al.; U.S. Pat. App. Pub. No. 2015/0335070 to Sears et al.; U.S. Pat. No. 6,204,287 to White; and U.S. Pat. No. 5,060,676 to Hearn et al., which are incorporated herein by reference in their entirety.

In some implementations, the substrate material may comprise a series of overlapping layers, as described in U.S. App. No. 16/196,958, filed on November, 20, 2018, and titled *Conductive Aerosol Generating Composite Substrate for Aerosol Source Member,* which is incorporated by reference herein in its entirety.

In some implementations, the amount of substrate material that is used within the smoking article may be such that the article exhibits acceptable sensory and organoleptic properties, and desirable performance characteristics. For example, in some implementations an aerosol precursor composition such as, for example, glycerin and/or propylene glycol, may be employed within the substrate material in order to provide for the generation of a visible mainstream aerosol that in many regards resembles the appearance of tobacco smoke. For example, the amount of aerosol precursor composition incorporated into the substrate material of the smoking article may be in the range of about 3.5 grams or less, about 3 grams or less, about 2.5 grams or less, about 2 grams or less, about 1.5 grams or less, about 1 gram or less, or about 0.5 gram or less.

According to another implementation, a smoking article according to the present disclosure may include a substrate material comprising a porous, inert material such as, for example, a ceramic material. For example, in some implementations ceramics of various shapes and geometries (e.g., beads, rods, tubes, etc.) may be used, which have various pore morphology. In addition, in some implementations non-tobacco materials, such as e-liquids, may be loaded into the ceramics. In another implementation, the substrate material may include a porous, inert material that does not substantially react, chemically and/or physically, with a tobacco-related material such as, for example, a tobacco-derived extract. In addition, an extruded tobacco, such as those described above, may be porous. For example, in some implementations an extruded tobacco material may have an inert gas, such as, for example, nitrogen, that acts as a blowing agent during the extrusion process.

As noted above, in various implementations the substrate material may include a tobacco, a tobacco component, and/or a tobacco-derived material that has been treated, manufactured, produced, and/or processed to incorporate an aerosol precursor composition (e.g., humectants such as, for example, propylene glycol, glycerin, and/or the like) and/or at least one flavoring agent, as well as a flame/burn retardant (e.g., diammonium phosphate and/or another salt) configured to help prevent ignition, pyrolysis, combustion, and/or scorching of the substrate material by the heat source. Various manners and methods for incorporating tobacco into smoking articles, and particularly smoking articles that are designed so as to not purposefully burn virtually all of the tobacco within those smoking articles are set forth in U.S. Pat. No. 4,947,874 to Brooks et al.; U.S. Pat. No. 7,647,932 to Cantrell et al.; U.S. Pat. No. 8,079,371 to Robinson et al.; U.S. Pat. No. 7,290,549 to Banerjee et al.; and U.S. Pat. App. Pub. No. 2007/0215167 to Crooks et al.; the disclosures of which are incorporated herein by reference in their entireties.

As noted, in some implementations, flame/burn retardant materials and other additives that may be included within the substrate material and may include organo-phosophorus compounds, borax, hydrated alumina, graphite, potassium tripolyphosphate, dipentaerythritol, pentaerythritol, and polyols. Others such as nitrogenous phosphonic acid salts, monoammonium phosphate, ammonium polyphosphate, ammonium bromide, ammonium borate, ethanolammonium borate, ammonium sulphamate, halogenated organic compounds, thiourea, and antimony oxides are suitable but are not preferred agents. In each aspect of flame-retardant, burn-retardant, and/or scorch-retardant materials used in the substrate material and/or other components (whether alone or in combination with each other and/or other materials), the desirable properties most preferably are provided without undesirable off-gassing or melting-type behavior.

According to other implementations of the present disclosure, the substrate material may also incorporate tobacco additives of the type that are traditionally used for the manufacture of tobacco products. Those additives may include the types of materials used to enhance the flavor and aroma of tobaccos used for the production of cigars, cigarettes, pipes, and the like. For example, those additives may include various cigarette casing and/or top dressing components. See, for example, U.S. Pat. No. 3,419,015 to Wochnowski; U.S. Pat. No. 4,054,145 to Berndt et al.; U.S. Pat. No. 4,887,619 to Burcham, Jr. et al.; U.S. Pat. No. 5,022,416 to Watson; U.S. Pat. No. 5,103,842 to Strang et al.; and U.S. Pat. No. 5,711,320 to Martin; the disclosures of which are incorporated herein by reference in their entireties. Preferred casing materials may include water, sugars and syrups (e.g., sucrose, glucose and high fructose corn syrup), humectants (e.g. glycerin or propylene glycol), and flavoring agents (e.g., cocoa and licorice). Those added components may also include top dressing materials (e.g., flavoring materials, such as menthol). See, for example, U.S. Pat. No. 4,449,541 to Mays et al., the disclosure of which is incorporated herein by reference in its entirety. Further materials that may be added include those disclosed in U.S. Pat. No. 4,830,028 to Lawson et al. and U.S. Pat. No. 8,186,360 to Marshall et al., the disclosures of which are incorporated herein by reference in their entireties.

As noted above, in various implementations, the substrate material may have an aerosol precursor composition associated therewith. For example, in some implementations the aerosol precursor composition may comprise one or more different components, such as polyhydric alcohol (e.g., glycerin, propylene glycol, or a mixture thereof). Representative types of further aerosol precursor compositions are set forth in U.S. Pat. No. 4,793,365 to Sensabaugh, Jr. et al.; U.S. Pat. No. 5,101,839 to Jakob et al.; PCT WO 98/57556 to Biggs et al.; and Chemical and Biological Studies on New Cigarette Prototypes that Heat Instead of Burn Tobacco, R. J. Reynolds Tobacco Company Monograph (1988); the disclosures of which are incorporated herein by reference. In some aspects, a substrate material may produce a visible aerosol upon the application of sufficient heat thereto (and cooling with air, if necessary), and the substrate material may produce an aerosol that is "smoke-like." In other aspects, the substrate material may produce an aerosol that is substantially non-visible but is recognized as present by other characteristics, such as flavor or texture. Thus, the nature of the produced aerosol may be variable depending upon the specific components of the aerosol delivery component. The substrate material may be chemically simple relative to the chemical nature of the smoke produced by burning tobacco.

A wide variety of types of flavoring agents, or materials that alter the sensory or organoleptic character or nature of the mainstream aerosol of the smoking article may be suitable to be employed. In some implementations, such flavoring agents may be provided from sources other than tobacco and may be natural or artificial (e.g., synthetic) in origin. For example, some flavoring agents may be applied to, or incorporated within, the substrate material and/or those regions of the smoking article where an aerosol is generated. In some implementations, such agents may be supplied directly to a heating cavity or region proximate to the heat source or are provided with the substrate material. Example flavoring agents may include, for example, vanillin, ethyl vanillin, cream, tea, coffee, fruit (e.g., apple, cherry, strawberry, peach and citrus flavors, including lime and lemon), maple, menthol, mint, peppermint, spearmint, wintergreen, nutmeg, clove, lavender, cardamom, ginger, honey, anise, sage, cinnamon, sandalwood, jasmine, cascarilla, cocoa, licorice, and flavorings and flavor packages of the type and character traditionally used for the flavoring of cigarette, cigar, and pipe tobaccos. Syrups, such as high fructose corn syrup, may also be suitable to be employed.

Flavoring agents may also include acidic or basic characteristics (e.g., organic acids, such as levulinic acid, succinic acid, pyruvic acid, and benzoic acid). In some implementations, flavoring agents may be combinable with the elements of the substrate material if desired. Example plant-derived compositions that may be suitable are disclosed in U.S. Pat. No. 9,107,453 and U.S. Pat. App. Pub. No. 2012/0152265 both to Dube et al., the disclosures of which are incorporated herein by reference in their entireties. Any of the materials, such as flavorings, casings, and the like that may be useful in combination with a tobacco material to affect sensory properties thereof, including organoleptic properties, such as described herein, may be combined with the substrate material. Organic acids particularly may be able to be incorporated into the substrate material to affect the flavor, sensation, or organoleptic properties of medicaments, such as nicotine, that may be able to be combined with the substrate material. For example, organic acids, such as levulinic acid, lactic acid, pyruvic acid, salicylic acid, sorbic acid, lauric acid, benzoic acid, and tartaric acid may be included in the substrate material with nicotine in amounts up to being equimolar (based on total organic acid content) with the nicotine. In alternative implementations, one or more acids may be included in amounts greater than equimolar amounts, such as, for example, to impart desired pH, sensory characteristics, etc. Any combination of organic acids may be suitable. For example, in some implementations, the substrate material may include approximately 0.1 to about 0.5 moles of levulinic acid per one mole of nicotine, approximately 0.1 to about 0.5 moles of pyruvic acid per one mole of nicotine, approximately 0.1 to about 0.5 moles of lactic acid per one mole of nicotine, or combinations thereof, up to a concentration wherein the total amount of organic acid present is equimolar to the total amount of nicotine present in the substrate material. Various additional examples of organic acids employed to produce a substrate material are described in U.S. Pat. App. Pub. No. 2015/0344456 to Dull et al., which is incorporated herein by reference in its entirety.

The selection of such further components may be variable based upon factors such as the sensory characteristics that are desired for the smoking article, and the present disclosure is intended to encompass any such further components that are readily apparent to those skilled in the art of tobacco and tobacco-related or tobacco-derived products. See, Gutcho, Tobacco Flavoring Substances and Methods, Noyes Data Corp. (1972) and Leffingwell et al., Tobacco Flavoring for Smoking Products (1972), the disclosures of which are incorporated herein by reference in their entireties.

In other implementations, the substrate material may include other materials having a variety of inherent characteristics or properties. For example, the substrate material may include a plasticized material or regenerated cellulose in the form of rayon. As another example, viscose (commercially available as VISIL^{®}), which is a regenerated cellulose product incorporating silica, may be suitable. Some carbon fibers may include at least 95 percent carbon or more. Similarly, natural cellulose fibers such as cotton may be suitable, and may be infused or otherwise treated with silica, carbon, or metallic particles to enhance flame-retardant properties and minimize off-gassing, particularly of any undesirable off-gassing components that would have a negative impact on flavor (and especially minimizing the likelihood of any toxic off-gassing products). Cotton may be treatable with, for example, boric acid or various organophosphate compounds to provide desirable flame-retardant properties by dipping, spraying or other techniques known in the art. These fibers may also be treatable (coated, infused, or both by, e.g., dipping, spraying, or vapor-deposition) with organic or metallic nanoparticles to confer the desired property of flame-retardancy without undesirable off-gassing or melting-type behavior.

Referring back to FIG. 4, at least a portion of the substrate portion **110** includes an overwrap material **142.** In the depicted implementation, the overwrap material **142** comprises an inner layer **144** positioned proximate an outer surface of the substrate material **140,** an intermediate layer **146** positioned proximate an outer surface of the inner layer **144,** and an outer layer **148** positioned proximate an outer surface of the intermediate layer **146.** In various implementations, the overwrap material **142** may be constructed as a laminate. In the depicted implementation, the inner layer **144** comprises an aerosol generating component that comprises an aerosol precursor composition. In some implementations, the inner layer **144** may be formed as a cast sheet or a reconstituted tobacco sheet that includes an aerosol precursor composition. As noted above, suitable aerosol precursor compositions may comprise, for example, propylene glycol, glycerin, and/or the like. In some implementations, the inner layer **144** may be constructed via a casting process, such as that described in U.S. Pat. No. 5,697,385 to Seymour et al., the disclosure of which is incorporated herein by reference in its entirety. For example, in some implementations a fibrous material, aerosol precursor composition, and binder may be blended together to form a slurry, which may be cast onto a surface (such as, for example, a moving belt). The cast slurry may then experience one or more drying and/or doctoring steps such that the result is a relatively consistent thickness cast sheet. Other examples of casting and paper-making techniques, are set forth in U.S. Pat. No. 4,674,519 to Keritsis et al.; U.S. Pat. No. 4,941,484 to Clapp et al.; U.S. Pat. No. 4,987,906 to Young et al.; U.S. Pat. No. 4,972,854 to Kiernan et al.; U.S. Pat. No. 5,099,864 to Young et al.; U.S. Pat. No. 5,143,097 to Sohn et al.; U.S. Pat. No. 5,159,942 to Brinkley et al.; U.S. Pat. No. 5,322,076 to Brinkley et al.; U.S. Pat. No. 5,339,838 to Young et al.; U.S. Pat. No. 5,377,698 to Litzinger et al.; U.S. Pat. No. 5,501,237 to Young; and U.S. Pat. No. 6,216,706 to Kumar; the disclosures of which is incorporated herein by reference in their entireties. Reference is also made to the discussion of coverings and fibrous materials provided in U.S. App. No. 16/196,958, filed on November 20, 2018, and titled *Conductive Aerosol Generating Composite Substrate for Aerosol Source Member,* which is incorporated by reference herein in its entirety.

In the depicted implementation, the aerosol generating component contained in the inner layer **144** has an aerosol former loading of approximately 2% - 20%. For example, although other configurations are possible in some implementations the inner layer may comprise a reconstituted tobacco sheet that comprises approximately 7% cellulose pulp, approximately 2% potassium carbonate, approximately 91% cut tobacco, and approximately 2 - 20% glycerin, wherein the tobacco percentage is adjusted to account for the glycerin percentage. In other configurations, the inner layer may comprise a cast sheet that comprises approximately 38% tobacco, approximately 6% ammonium alginate, approximately 4% ammonium phosphate, and approximately 2 - 20% glycerin, wherein the tobacco percentage is adjusted to account for the glycerin percentage. Although other configurations are possible, in various implementations, the thickness of the inner layer may be approximately 0.1 mm - 0.5 mm, and in some implementations, 0.1 mm - 0.5 mm.

As noted, the overwrap material **142** also includes an intermediate layer **146** and an outer layer **148.** In the depicted implementation, the intermediate layer **146** comprises a foil material, such as, for example, a metal foil material, such as an aluminum foil material. In other implementations, the intermediate layer **146** may comprise other materials, including, but not limited to, a copper material, a tin material, a gold material, a graphene material, a graphite material or other thermally conductive carbon-based material, and/or any combinations thereof. Although other configurations are possible, in some implementations the intermediate layer may comprise an aluminum foil material having a thickness from 15 microns - 5 mm. In the depicted implementation the outer layer **148** comprises a paper layer, such as a conventional cigarette wrapping paper. In some implementations, the paper material may comprise rag fibers, such as non-wood plant fibers, and may include flax, hemp, sisal, rice straw, and/or esparto fibers. Although other configurations are possible, in some implementations the outer layer may comprise a paper material having a thickness of approximately 0.03 mm - 0.5 mm with a weight of approximately 18 - 30 gsm. In other implementations, the outer layer may comprise other materials, including, for example a wire mesh.

In various implementations, the inhalable substance generated by the application of heat to the substrate material **140** produces a primary inhalable substance. The present disclosure also provides that another inhalable substance may be generated by the application of heat to the overwrap material **142.** For example, in the depicted implementation, in which the heating member **132** substantially surrounds an outside surface of the substrate portion **110** and heats the substrate portion **110** from the outside inward (e.g., the heating member **132** is a circumferential heating member located proximate the overwrap material **142),** an initial inhalable substance may be generated by the application of heat to the overwrap material **142.** In such a manner, upon activation of the circumferential heating member **132** and heating of the substrate portion **110** of the aerosol source member **102,** an initial inhalable substance is generated from the overwrap material **142,** and then, later in the heating cycle, a primary inhalable substance is generated from the substrate material **140.** It should be noted that various other types of heating members or combinations of heating members may be used that heat the substrate portion, at least partially, from the outside inward. For example, in some implementations the heating member need not be a circumferential heating member, but, rather, may comprise one or more components that engage a portion of the surface of the substrate portion (e.g., a spiral heating member, or a heating member that includes one or more fins or protuberances).

It should be noted that in other implementations, a control body may provide a heating element that heats from the inside of an aerosol source member. In such implementations, application of heat to the overwrap material **142** may produce a secondary inhalable substance. In such a manner, upon activation of an internally located heating member and heating of the substrate portion of the aerosol source member, a primary inhalable substance is generated from the substrate material, and then, later in the heating cycle, a secondary inhalable substance is generated from the overwrap material. Some examples of internally located heating members are described in U.S. Pat. App. No. 15/916,834, to Sur et al., U.S. Pat. No. 9,078,473 to Worm et al., and U.S. Pat. No. 7,726,320 to Robinson et al., each of which is incorporated herein by reference in its entirety.

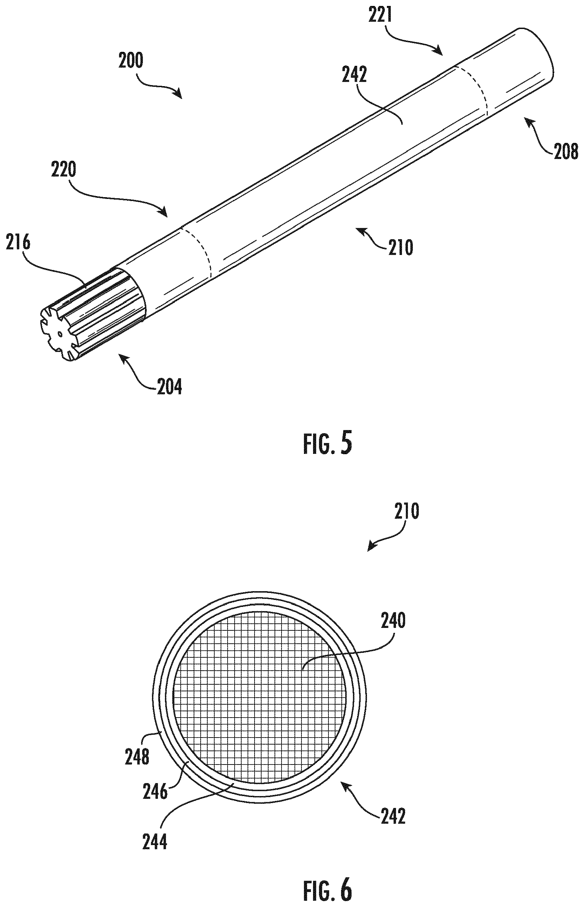

FIG. 5 illustrates a perspective view of an aerosol source member, according to an example implementation of the present disclosure. In particular, FIG. 5 illustrates an aerosol source member **200.** In the depicted implementation, the aerosol source member **200** includes a heat source **204** and a substrate portion **210.** A mouth end **208** is defined opposite the heat source **204.** Although an aerosol delivery device and/or an aerosol source member according to the present disclosure may take on a variety of implementations, as discussed in detail below, the use of the aerosol delivery device and/or aerosol source member by a consumer will be similar in scope. The foregoing description of use of the aerosol source member is applicable to the various implementations described through minor modifications, which are apparent to the person of skill in the art in light of the further disclosure provided herein. The description of use, however, is not intended to limit the use of the articles of the present disclosure but is provided to comply with all necessary requirements of disclosure herein.

In various implementations, the heat source **204** may be configured to generate heat upon ignition thereof. In the depicted implementation, the heat source **204** comprises a combustible fuel element that has a generally cylindrical shape and that incorporates a combustible carbonaceous material. In other implementations, the heat source **204** may have a different shape, for example, a prism shape having a cubic or hexagonal cross-section. Carbonaceous materials generally have a high carbon content. Preferred carbonaceous materials may be composed predominately of carbon, and/or typically may have carbon contents of greater than about 60 percent, generally greater than about 70 percent, often greater than about 80 percent, and frequently greater than about 90 percent, on a dry weight basis. It should be noted that in other implementations, other types of heat sources are possible, including a heat source that comprises a combustible liquid fuel.

In some instances, the heat source **204** may incorporate elements other than combustible carbonaceous materials (e.g., tobacco components, such as powdered tobaccos or tobacco extracts; flavoring agents; salts, such as sodium chloride, potassium chloride and sodium carbonate; heat stable graphite fibers; iron oxide powder; glass filaments; powdered calcium carbonate; alumina granules; ammonia sources, such as ammonia salts; and/or binding agents, such as guar gum, ammonium alginate and sodium alginate). Although specific dimensions of an applicable heat source may vary, in some implementations, the heat source **204** may have a length in an inclusive range of approximately 7 mm to approximately 20 mm, and in some implementations may be approximately 17 mm, and an overall diameter in an inclusive range of approximately 3 mm to approximately 8 mm, and in some implementations may be approximately 4.8 mm (and in some implementations, approximately 7 mm). Although in other implementations, the heat source may be constructed in a variety of ways, in the depicted implementation, the heat source **204** is extruded or compounded using a ground or powdered carbonaceous material, and has a density that is greater than about 0.5 g/cm³, often greater than about 0.7 g/cm³, and frequently greater than about 1 g/cm³, on a dry weight basis. See, for example, the types of fuel source components, formulations and designs set forth in U.S. Pat. No. 5,551,451 to Riggs et al. and U.S. Pat. No. 7,836,897 to Borschke et al., which are incorporated herein by reference in their entireties. Although in various implementations, the heat source may have a variety of forms, including, for example, a substantially solid cylindrical shape or a hollow cylindrical (e.g., tube) shape, the heat source **204** of the depicted implementation comprises an extruded monolithic carbonaceous material that has a generally cylindrical shape but with a plurality of grooves **216** extending longitudinally from a first end of the extruded monolithic carbonaceous material to an opposing second end of the extruded monolithic carbonaceous material. In some implementations, the aerosol source member, and in particular, the heat source may include a heat transfer component. In various implementations, a heat transfer component may be proximate the heat source, and, in some implementations, a heat transfer component may be located in or within the heat source. Some examples of heat transfer components are described in in U.S. Pat. App. No. 15/923,735, filed on March 16, 2018, and titled *Smoking Article with Heat Transfer Component,* which is incorporated herein by reference in its entirety. Although in the depicted implementation, the grooves **216** of the heat source **204** are substantially equal in width and depth and are substantially equally distributed about a circumference of the heat source **204,** other implementations may include as few as two grooves, and still other implementations may include as few as a single groove. Still other implementations may include no grooves at all. Additional implementations may include multiple grooves that may be of unequal width and/or depth, and which may be unequally spaced around a circumference of the heat source. In still other implementations, the heat source may include flutes and/or slits extending longitudinally from a first end of the extruded monolithic carbonaceous material to an opposing second end thereof. In some implementations, the heat source may comprise a foamed carbon monolith formed in a foam process of the type disclosed in U.S. Pat. No. 7,615,184 to Lobovsky, which is incorporated herein by reference in its entirety. As such, some implementations may provide advantages with regard to reduced time taken to ignite the heat source. In some other implementations, the heat source may be co-extruded with a layer of insulation (not shown), thereby reducing manufacturing time and expense. Other implementations of fuel elements include carbon fibers of the type described in U.S. Pat. No. 4,922,901 to Brooks et al. or other heat source implementations such as is disclosed in U.S. Pat. App. Pub. No. 2009/0044818 to Takeuchi et al., each of which is incorporated herein by reference in its entirety.

Generally, the heat source is positioned sufficiently near a substrate portion (e.g., an aerosol delivery component) having one or more aerosolizable components so that the aerosol formed/volatilized by the application of heat from the heat source to the aerosolizable components (as well as any flavorants, medicaments, and/or the like that are likewise provided for delivery to a user) is deliverable to the user by way of the mouthpiece. That is, when the heat source heats the substrate portion, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof. Additionally, the selection of various aerosol delivery device elements are appreciated upon consideration of commercially available electronic aerosol delivery devices, such as those representative products listed in the background art section of the present disclosure.

As will be discussed in more detail below, an overwrap material **242** may be provided that surrounds at least a portion of the substrate portion **210.** In the depicted implementation, the overwrap material **242** engages or otherwise joins together at least a portion of the heat source **204** with the substrate portion **210.** As shown in FIG. 5, the overwrap material **242** may also include one or more openings **220** formed therethrough that allow the entry of air upon a draw on the mouth end **208.** In various implementations, the size and number of these openings may vary based on particular design requirements. In the depicted implementation, a plurality of openings **220** are located proximate an end of the substrate portion **210** closest to the heat source **204,** and a plurality of separate cooling openings **221** may be formed in the overwrap material **242** in an area proximate the mouth end **208.** Although other implementations may differ, in the depicted implementation, the openings **220** comprise a plurality of openings substantially evenly spaced about the outer surface of the aerosol source member **200,** and the openings **221** also comprise a plurality of openings substantially evenly spaced around the outer surface of the aerosol source member **200.** Although in various implementations the plurality of openings may be formed through the overwrap material **242** in a variety of ways, in the depicted implementation, the plurality of openings **220** and the plurality of separate cooling openings **221** are formed via laser perforation.

In the depicted implementation, the aerosol source member **200** includes a substrate portion **210** having opposed first and second ends, wherein the first end is disposed proximate the heat source **204.** Although the depicted implementation only includes one substrate portion, other implementations may include separate substrate portions, such as a second substrate portion disposed proximate the second end of substrate portion **210.** In other implementations, additional substrate portions may be included.

In various implementations, the mouth end **208** of the aerosol source member **200** may include a filter, which may, for example, be made of a cellulose acetate or polypropylene material. The filter may additionally or alternatively contain strands of tobacco containing material, such as described in U.S. Pat. No. 5,025,814 to Raker et al., which is incorporated herein by reference in its entirety. In various implementations, the filter may increase the structural integrity of the mouth end of the aerosol source member, and/or provide filtering capacity, if desired, and/or provide resistance to draw. In some implementations, the filter may comprise discrete segments. For example, some implementations may include a segment providing filtering, a segment providing draw resistance, a hollow segment providing a space for the aerosol to cool, a segment providing increased structural integrity, other filter segments, and any one or any combination of the above.

In various implementations, other components may exist between the substrate portion **210** and the mouth end **208** of the aerosol source member **200.** For example, in some implementations one or any combination of the following may be positioned between the substrate portion **210** and the mouth end **208** of the aerosol source member **200:** an air gap; a hollow tube structure; phase change materials for cooling air; flavor releasing media; ion exchange fibers capable of selective chemical adsorption; aerogel particles as filter medium; and other suitable materials. Some examples of possible phase change materials include, but are limited to, salts, such as AgNO₃, AlCl₃, TaCl₃, InCl₃, SnCl₂, AlI₃, and TiI₄; metals and metal alloys such as selenium, tin, indium, tin-zinc, indium-zinc, or indium-bismuth; and organic compounds such as D-mannitol, succinic acid, p-nitrobenzoic acid, hydroquinone and adipic acid. Other examples are described in U.S. Pat. No. 8,430,106 to Potter et al., which is incorporated herein by reference in its entirety.

FIG. 6 illustrates a cross-section schematic view of a portion of the aerosol source member of FIG. 5, according to an example implementation of the present disclosure. In particular, FIG. 6 illustrates a portion of the substrate portion **210** of the aerosol source member **200,** which comprises a substrate material **240** substantially surrounded by an overwrap material **242.** In various implementations the substrate material **240** is configured to generate an inhalable substance upon the application of heat thereupon, and may, or may not, include tobacco derived materials. In one implementation, the substrate material may comprise a blend of flavorful and aromatic tobaccos in cut filler form. In another implementation, the substrate material may comprise a reconstituted tobacco material, such as described in U.S. Pat. No. 4,807,809 to Pryor et al.; U.S. Pat. No. 4,889,143 to Pryor et al. and U.S. Pat. No. 5,025,814 to Raker, the disclosures of which are incorporated herein by reference in their entirety. Additionally, a reconstituted tobacco material may include a reconstituted tobacco paper for the type of cigarettes described in Chemical and Biological Studies on New Cigarette Prototypes that Heat Instead of Burn Tobacco, R. J. Reynolds Tobacco Company Monograph (1988), the contents of which are incorporated herein by reference in its entirety. For example, a reconstituted tobacco material may include a sheet-like material containing tobacco and/or tobacco-related materials. As such, in some implementations, the substrate material may be formed from a wound roll of a reconstituted tobacco material. In another implementation, the substrate material may be formed from shreds, strips, and/or the like of a reconstituted tobacco material. In another implementation, the tobacco sheet may comprise overlapping layers (e.g., a gathered web), which may, or may not, include heat conducting constituents. Examples of substrate portions that include a series of overlapping layers (e.g., gathered webs) of an initial substrate sheet formed by the fibrous filler material, aerosol forming material, and plurality of heat conducting constituents are described in U.S. Pat. App. No. 15/905,320, filed on February 26, 2018, and titled *Heat Conducting Substrate for Electrically Heated Aerosol Delivery Device,* which is incorporated herein by reference in its entirety.

In some implementations, the substrate material may include a plurality of microcapsules, beads, granules, and/or the like having a tobacco-related material. For example, a representative microcapsule may be generally spherical in shape, and may have an outer cover or shell that contains a liquid center region of a tobacco-derived extract and/or the like. In some implementations, the substrate material may include a plurality of microcapsules each formed into a hollow cylindrical shape. In some implementations, the substrate material may include a binder material configured to maintain the structural shape and/or integrity of the plurality of microcapsules formed into the hollow cylindrical shape.

Tobacco employed in the substrate material can vary and may include, for example, flue-cured tobacco, burley tobacco, Oriental tobacco or Maryland tobacco, dark tobacco, dark-fired tobacco and *Rustica* tobaccos, as well as other rare or specialty tobaccos, or blends thereof. Tobacco materials also can include so-called "blended" forms and processed forms, such as processed tobacco stems (e.g., cut-rolled or cut-puffed stems), volume expanded tobacco (e.g., puffed tobacco, such as dry ice expanded tobacco (DIET), preferably in cut filler form), reconstituted tobaccos (e.g., reconstituted tobaccos manufactured using paper-making type or cast sheet type processes). Various representative tobacco types, processed types of tobaccos, and types of tobacco blends are set forth in U.S. Pat. Nos. 4,836,224 to Lawson et al.; 4,924,888 to Perfetti et al.; 5,056,537 to Brown et al.; 5,159,942 to Brinkley et al.; 5,220,930 to Gentry; 5,360,023 to Blakley et al.; 6,701,936 to Shafer et al.; 7,011,096 to Li et al.; and 7,017,585 to Li et al.; 7,025,066 to Lawson et al.; U.S. Pat. App. Pub. No. 2004-0255965 to Perfetti et al.; PCT Pat. App. Pub. No. WO 02/37990 to Bereman; and Bombick et al., Fund. Appl. Toxicol., 39, p. 11-17 (1997); which are incorporated herein by reference in their entireties. Further examples of tobacco compositions that may be useful are disclosed in U.S. Pat. No. 7,726,320 to Robinson et al., which is incorporated herein by reference in its entirety. In some implementations, the milled tobacco material may comprise a blend of flavorful and aromatic tobaccos. In another implementation, the tobacco material may comprise a reconstituted tobacco material, such as described in U.S. Pat. No. 4,807,809 to Pryor et al.; U.S. Pat. No. 4,889,143 to Pryor et al. and U.S. Pat. No. 5,025,814 to Raker, the disclosures of which are incorporated herein by reference in their entirety. Additionally, a reconstituted tobacco material may include a reconstituted tobacco paper for the type of cigarettes described in Chemical and Biological Studies on New Cigarette Prototypes that Heat Instead of Burn Tobacco, R. J. Reynolds Tobacco Company Monograph (1988), the contents of which are incorporated herein by reference in its entirety.

In still other implementations of the present disclosure, the substrate material may include an extruded structure that includes, or is essentially comprised of a tobacco, a tobacco related material, glycerin, water, and/or a binder material, although certain formulations may exclude the binder material. In various implementations, suitable binder materials may include alginates, such as ammonium alginate, propylene glycol alginate, potassium alginate, and sodium alginate. Alginates, and particularly high viscosity alginates, may be employed in conjunction with controlled levels of free calcium ions. Other suitable binder materials include hydroxypropylcellulose such as Klucel H from Aqualon Co.; hydroxypropylmethylcellulose such as Methocel K4MS from The Dow Chemical Co.; hydroxyethylcellulose such as Natrosol 250 MRCS from Aqualon Co.; microcrystalline cellulose such as Avicel from FMC; methylcellulose such as Methocel A4M from The Dow Chemical Co.; and sodium carboxymethyl cellulose such as CMC 7HF and CMC 7H4F from Hercules Inc. Still other possible binder materials include starches (e.g., corn starch), guar gum, carrageenan, locust bean gum, pectins and xanthan gum. In some implementations, combinations or blends of two or more binder materials may be employed. Other examples of binder materials are described, for example, in U.S. Pat. No. 5,101,839 to Jakob et al.; and U.S. Pat. No. 4,924,887 to Raker et al., each of which is incorporated herein by reference in its entirety. In some implementations, the aerosol forming material may be provided as a portion of the binder material (e.g., propylene glycol alginate). In addition, in some implementations, the binder material may comprise nanocellulose derived from a tobacco or other biomass.

In some implementations, the substrate material may include an extruded material, as described in U.S. Pat. App. Pub. No. 2012/0042885 to Stone et al., which is incorporated herein by reference in its entirety. In yet another implementation, the substrate material may include an extruded structure and/or substrate formed from marumarized and/or non-marumarized tobacco. Marumarized tobacco is known, for example, from U.S. Pat. No. 5,105,831 to Banerjee, et al., which is incorporated by reference herein in its entirety. Marumarized tobacco includes about 20 to about 50 percent (by weight) tobacco blend in powder form, with glycerol (at about 20 to about 30 percent weight), calcium carbonate (generally at about 10 to about 60 percent by weight, often at about 40 to about 60 percent by weight), along with binder agents, as described herein, and/or flavoring agents. In various implementations, the extruded material may have one or more longitudinal openings.

In various implementations, the substrate material may take on a variety of conformations based upon the various amounts of materials utilized therein. For example, a sample substrate material may comprise up to approximately 98% by weight, up to approximately 95% by weight, or up to approximately 90% by weight of a tobacco and/or tobacco related material. A sample substrate material may also comprise up to approximately 25% by weight, approximately 20% by weight, or approximately 15% by weight water - particularly approximately 2% to approximately 25%, approximately 5% to approximately 20%, or approximately 7% to approximately 15% by weight water. Flavors and the like (which include, for example, medicaments, such as nicotine) may comprise up to approximately 10%, up to about 8%, or up to about 5% by weight of the aerosol delivery component.

Additionally or alternatively, the substrate material may include an extruded structure and/or a substrate that includes or essentially is comprised of tobacco, glycerin, water, and/or binder material, and is further configured to substantially maintain its structure throughout the aerosol-generating process. That is, the substrate material may be configured to substantially maintain its shape (e.g., the substrate material does not continually deform under an applied shear stress) throughout the aerosol-generating process. Although such an example substrate material may include liquids and/or some moisture content, the substrate may remain substantially solid throughout the aerosol-generating process and may substantially maintain structural integrity throughout the aerosol-generating process. Example tobacco and/or tobacco related materials suitable for a substantially solid substrate material are described in U.S. Pat. App. Pub. No. 2015/0157052 to Ademe et al.; U.S. Pat. App. Pub. No. 2015/0335070 to Sears et al.; U.S. Pat. No. 6,204,287 to White; and U.S. Pat. No. 5,060,676 to Hearn et al., which are incorporated herein by reference in their entirety.

In some implementations, the substrate material may comprise a series of overlapping layers, as described in U.S. App. No. 16/196,958, filed on November 20, 2018, and titled *Conductive Aerosol Generating Composite Substrate for Aerosol Source Member,* which is incorporated by reference herein in its entirety.

In some implementations, the amount of substrate material that is used within the smoking article may be such that the article exhibits acceptable sensory and organoleptic properties, and desirable performance characteristics. For example, in some implementations an aerosol precursor composition such as, for example, glycerin and/or propylene glycol, may be employed within the substrate material in order to provide for the generation of a visible mainstream aerosol that in many regards resembles the appearance of tobacco smoke. For example, the amount of aerosol precursor composition incorporated into the substrate material of the smoking article may be in the range of about 3.5 grams or less, about 3 grams or less, about 2.5 grams or less, about 2 grams or less, about 1.5 grams or less, about 1 gram or less, or about 0.5 gram or less.

According to another implementation, a smoking article according to the present disclosure may include a substrate material comprising a porous, inert material such as, for example, a ceramic material. For example, in some implementations ceramics of various shapes and geometries (e.g., beads, rods, tubes, etc.) may be used, which have various pore morphology. In addition, in some implementations non-tobacco materials, such as e-liquids, may be loaded into the ceramics. In another implementation, the substrate material may include a porous, inert material that does not substantially react, chemically and/or physically, with a tobacco-related material such as, for example, a tobacco-derived extract. In addition, an extruded tobacco, such as those described above, may be porous. For example, in some implementations an extruded tobacco material may have an inert gas, such as, for example, nitrogen, that acts as a blowing agent during the extrusion process.

As noted above, in various implementations the substrate material may include a tobacco, a tobacco component, and/or a tobacco-derived material that has been treated, manufactured, produced, and/or processed to incorporate an aerosol precursor composition (e.g., humectants such as, for example, propylene glycol, glycerin, and/or the like) and/or at least one flavoring agent, as well as a flame/burn retardant (e.g., diammonium phosphate and/or another salt) configured to help prevent ignition, pyrolysis, combustion, and/or scorching of the substrate material by the heat source. Various manners and methods for incorporating tobacco into smoking articles, and particularly smoking articles that are designed so as to not purposefully burn virtually all of the tobacco within those smoking articles are set forth in U.S. Pat. No. 4,947,874 to Brooks et al.; U.S. Pat. No. 7,647,932 to Cantrell et al.; U.S. Pat. No. 8,079,371 to Robinson et al.; U.S. Pat. No. 7,290,549 to Banerjee et al.; and U.S. Pat. App. Pub. No. 2007/0215167 to Crooks et al.; the disclosures of which are incorporated herein by reference in their entireties.

As noted, in some implementations, flame/burn retardant materials and other additives that may be included within the substrate material and may include organo-phosophorus compounds, borax, hydrated alumina, graphite, potassium tripolyphosphate, dipentaerythritol, pentaerythritol, and polyols. Others such as nitrogenous phosphonic acid salts, monoammonium phosphate, ammonium polyphosphate, ammonium bromide, ammonium borate, ethanolammonium borate, ammonium sulphamate, halogenated organic compounds, thiourea, and antimony oxides are suitable but are not preferred agents. In each aspect of flame-retardant, burn-retardant, and/or scorch-retardant materials used in the substrate material and/or other components (whether alone or in combination with each other and/or other materials), the desirable properties most preferably are provided without undesirable off-gassing or melting-type behavior.

According to other implementations of the present disclosure, the substrate material may also incorporate tobacco additives of the type that are traditionally used for the manufacture of tobacco products. Those additives may include the types of materials used to enhance the flavor and aroma of tobaccos used for the production of cigars, cigarettes, pipes, and the like. For example, those additives may include various cigarette casing and/or top dressing components. See, for example, U.S. Pat. No. 3,419,015 to Wochnowski; U.S. Pat. No. 4,054,145 to Berndt et al.; U.S. Pat. No. 4,887,619 to Burcham, Jr. et al.; U.S. Pat. No. 5,022,416 to Watson; U.S. Pat. No. 5,103,842 to Strang et al.; and U.S. Pat. No. 5,711,320 to Martin; the disclosures of which are incorporated herein by reference in their entireties. Preferred casing materials may include water, sugars and syrups (e.g., sucrose, glucose and high fructose corn syrup), humectants (e.g. glycerin or propylene glycol), and flavoring agents (e.g., cocoa and licorice). Those added components may also include top dressing materials (e.g., flavoring materials, such as menthol). See, for example, U.S. Pat. No. 4,449,541 to Mays et al., the disclosure of which is incorporated herein by reference in its entirety. Further materials that may be added include those disclosed in U.S. Pat. No. 4,830,028 to Lawson et al. and U.S. Pat. No. 8,186,360 to Marshall et al., the disclosures of which are incorporated herein by reference in their entireties.

As noted above, in various implementations, the substrate material may have an aerosol precursor composition associated therewith. For example, in some implementations the aerosol precursor composition may comprise one or more different components, such as polyhydric alcohol (e.g., glycerin, propylene glycol, or a mixture thereof). Representative types of further aerosol precursor compositions are set forth in U.S. Pat. No. 4,793,365 to Sensabaugh, Jr. et al.; U.S. Pat. No. 5,101,839 to Jakob et al.; PCT WO 98/57556 to Biggs et al.; and Chemical and Biological Studies on New Cigarette Prototypes that Heat Instead of Burn Tobacco, R. J. Reynolds Tobacco Company Monograph (1988); the disclosures of which are incorporated herein by reference. In some aspects, a substrate material may produce a visible aerosol upon the application of sufficient heat thereto (and cooling with air, if necessary), and the substrate material may produce an aerosol that is "smoke-like." In other aspects, the substrate material may produce an aerosol that is substantially non-visible but is recognized as present by other characteristics, such as flavor or texture. Thus, the nature of the produced aerosol may be variable depending upon the specific components of the aerosol delivery component. The substrate material may be chemically simple relative to the chemical nature of the smoke produced by burning tobacco.

A wide variety of types of flavoring agents, or materials that alter the sensory or organoleptic character or nature of the mainstream aerosol of the smoking article may be suitable to be employed. In some implementations, such flavoring agents may be provided from sources other than tobacco and may be natural or artificial (e.g., synthetic) in origin. For example, some flavoring agents may be applied to, or incorporated within, the substrate material and/or those regions of the smoking article where an aerosol is generated. In some implementations, such agents may be supplied directly to a heating cavity or region proximate to the heat source or are provided with the substrate material. Example flavoring agents may include, for example, vanillin, ethyl vanillin, cream, tea, coffee, fruit (e.g., apple, cherry, strawberry, peach and citrus flavors, including lime and lemon), maple, menthol, mint, peppermint, spearmint, wintergreen, nutmeg, clove, lavender, cardamom, ginger, honey, anise, sage, cinnamon, sandalwood, jasmine, cascarilla, cocoa, licorice, and flavorings and flavor packages of the type and character traditionally used for the flavoring of cigarette, cigar, and pipe tobaccos. Syrups, such as high fructose corn syrup, may also be suitable to be employed.

Flavoring agents may also include acidic or basic characteristics (e.g., organic acids, such as levulinic acid, succinic acid, pyruvic acid, and benzoic acid). In some implementations, flavoring agents may be combinable with the elements of the substrate material if desired. Example plant-derived compositions that may be suitable are disclosed in U.S. Pat. No. 9,107,453 and U.S. Pat. App. Pub. No. 2012/0152265 both to Dube et al., the disclosures of which are incorporated herein by reference in their entireties. Any of the materials, such as flavorings, casings, and the like that may be useful in combination with a tobacco material to affect sensory properties thereof, including organoleptic properties, such as described herein, may be combined with the substrate material. Organic acids particularly may be able to be incorporated into the substrate material to affect the flavor, sensation, or organoleptic properties of medicaments, such as nicotine, that may be able to be combined with the substrate material. For example, organic acids, such as levulinic acid, lactic acid, and pyruvic acid, may be included in the substrate material with nicotine in amounts up to being equimolar (based on total organic acid content) with the nicotine. Any combination of organic acids may be suitable. For example, in some implementations, the substrate material may include approximately 0.1 to about 0.5 moles of levulinic acid per one mole of nicotine, approximately 0.1 to about 0.5 moles of pyruvic acid per one mole of nicotine, approximately 0.1 to about 0.5 moles of lactic acid per one mole of nicotine, or combinations thereof, up to a concentration wherein the total amount of organic acid present is equimolar to the total amount of nicotine present in the substrate material. Various additional examples of organic acids employed to produce a substrate material are described in U.S. Pat. App. Pub. No. 2015/0344456 to Dull et al., which is incorporated herein by reference in its entirety.

The selection of such further components may be variable based upon factors such as the sensory characteristics that are desired for the smoking article, and the present disclosure is intended to encompass any such further components that are readily apparent to those skilled in the art of tobacco and tobacco-related or tobacco-derived products. See, Gutcho, Tobacco Flavoring Substances and Methods, Noyes Data Corp. (1972) and Leffingwell et al., Tobacco Flavoring for Smoking Products (1972), the disclosures of which are incorporated herein by reference in their entireties.

In other implementations, the substrate material may include other materials having a variety of inherent characteristics or properties. For example, the substrate material may include a plasticized material or regenerated cellulose in the form of rayon. As another example, viscose (commercially available as VISIL^{®}), which is a regenerated cellulose product incorporating silica, may be suitable. Some carbon fibers may include at least 95 percent carbon or more. Similarly, natural cellulose fibers such as cotton may be suitable, and may be infused or otherwise treated with silica, carbon, or metallic particles to enhance flame-retardant properties and minimize off-gassing, particularly of any undesirable off-gassing components that would have a negative impact on flavor (and especially minimizing the likelihood of any toxic off-gassing products). Cotton may be treatable with, for example, boric acid or various organophosphate compounds to provide desirable flame-retardant properties by dipping, spraying or other techniques known in the art. These fibers may also be treatable (coated, infused, or both by, e.g., dipping, spraying, or vapor-deposition) with organic or metallic nanoparticles to confer the desired property of flame-retardancy without undesirable off-gassing or melting-type behavior.

With reference to FIG. 6, the overwrap material **242** of the depicted implementation comprises an inner layer **244** positioned proximate an outer surface of the substrate material **240,** an intermediate layer **246** positioned proximate an outer surface of the inner layer **244,** and an outer layer **248** positioned proximate an outer surface of the intermediate layer **246.** In various implementations, the overwrap material **242** may be constructed as a laminate. In the depicted implementation, the inner layer **244** comprises an aerosol generating component that comprises an aerosol precursor composition. In some implementations, the inner layer **244** may be formed as a cast sheet or a reconstituted tobacco sheet that includes an aerosol precursor composition. As noted above, suitable aerosol precursor compositions may comprise, for example, propylene glycol, glycerin, and/or the like. In some implementations the inner layer **244** may be constructed via a casting process, such as that described in U.S. Pat. No. 5,697,385 to Seymour et al., the disclosure of which is incorporated herein by reference in its entirety. For example, in some implementations a fibrous material, aerosol precursor composition, and binder may be blended together to form a slurry, which may be cast onto a surface (such as, for example, a moving belt). The cast slurry may then experience one or more drying and/or doctoring steps such that the result is a relatively consistent thickness cast sheet. Other examples of casting and paper-making techniques, are set forth in U.S. Pat. No. 4,674,519 to Keritsis et al.; U.S. Pat. No. 4,941,484 to Clapp et al.; U.S. Pat. No. 4,987,906 to Young et al.; U.S. Pat. No. 4,972,854 to Kiernan et al.; U.S. Pat. No. 5,099,864 to Young et al.; U.S. Pat. No. 5,143,097 to Sohn et al.; U.S. Pat. No. 5,159,942 to Brinkley et al.; U.S. Pat. No. 5,322,076 to Brinkley et al.; U.S. Pat. No. 5,339,838 to Young et al.; U.S. Pat. No. 5,377,698 to Litzinger et al.; U.S. Pat. No. 5,501,237 to Young; and U.S. Pat. No. 6,216,706 to Kumar; the disclosures of which is incorporated herein by reference in their entireties. Reference is also made to the discussion of coverings and fibrous materials provided in U.S. App. No. 16/196,958, filed on November 20, 2018, and titled *Conductive Aerosol Generating Composite Substrate for Aerosol Source Member,* which is incorporated by reference herein in its entirety. Further reference is made to the discussions of cast sheets and reconstituted tobacco sheets above.

In the depicted implementation, the aerosol generating component contained in the inner layer **244** has an aerosol former loading of approximately 2% - 20%. For example, although other configurations are possible in some implementations the inner layer may comprise a reconstituted tobacco sheet that comprises approximately 7% cellulose pulp, approximately 2% potassium carbonate, approximately 91% cut tobacco, and approximately 2 - 20% glycerin, wherein the tobacco percentage is adjusted to account for the glycerin percentage. In other configurations, the inner layer may comprise a cast sheet that comprises approximately 38% tobacco, approximately 6% ammonium alginate, approximately 4% ammonium phosphate, and approximately 2 - 20% glycerin, wherein the tobacco percentage is adjusted to account for the glycerin percentage. Although other configurations are possible, in various implementations, the thickness of the inner layer may be approximately 0.1 mm - 0.5 mm, and in some implementations, 0.1 mm - 0.5 mm.

As noted, the overwrap material **242** also includes an intermediate layer **246** and an outer layer **248.** In the depicted implementation, the intermediate layer **246** comprises a foil material, such as, for example, a metal foil material, such as an aluminum foil material. In other implementations, the intermediate layer **246** may comprise other materials, including, but not limited to, a copper material, a tin material, a gold material, a graphene material, a graphite material or other thermally conductive carbon-based material, and/or any combinations thereof. Although other configurations are possible, in some implementations the intermediate layer may comprise an aluminum foil material having a thickness from 15 microns - 5 mm. In the depicted implementation the outer layer **248** comprises a paper layer, such as a conventional cigarette wrapping paper. In various implementations, the paper material may comprise rag fibers, such as non-wood plant fibers, and may include flax, hemp, sisal, rice straw, and/or esparto fibers. Although other configurations are possible, in some implementations the outer layer may comprise a paper material having a thickness of approximately 0.03 mm - 0.5 mm and a weight of approximately 18 - 30 gsm.

In various implementations, the inhalable substance generated by the application of heat to the substrate material **240** may produce a primary inhalable substance. The present disclosure also provides that another inhalable substance may be generated by the application of heat to the overwrap material **242.** In some implementations, the carbon-based heat source may heat both radially as well as tangentially. In the depicted implementation, in which the carbon-based heat source heats from the inside outward, a secondary inhalable substance may be generated by the application of heat to the overwrap material **242.** In such a manner, upon ignition of the carbon heat source **204** and heating of the substrate portion **210** of the aerosol source member **202,** a primary inhalable substance is generated from the substrate material **240,** and then, later in the heating cycle, a secondary inhalable substance is generated from the overwrap material **242.**

It should be noted that in other implementations, in which a carbon-based heat source heats from the outside of the substrate portion inward, the inhalable substance generated by the application of heat to the overwrap material may produce an initial inhalable substance. In such a manner, upon ignition of the carbon-based heat source and heating of the substrate portion, an initial inhalable substance is generated from the overwrap material, and then, later in the heating cycle, a secondary inhalable substance is generated from the substrate material. In still other implementations, the overwrap material may produce both an initial inhalable substance and a secondary inhalable substance.

Although in some implementations an aerosol source member and a control body may be provided together as a complete smoking article or pharmaceutical delivery article generally, the components may be provided separately. For example, the present disclosure also encompasses a disposable unit for use with a reusable smoking article or a reusable pharmaceutical delivery article. In specific implementations, such a disposable unit (which may be an aerosol source member as illustrated in the appended figures) can comprise a substantially tubular shaped body having a heated end configured to engage the reusable smoking article or pharmaceutical delivery article, an opposing mouth end configured to allow passage of an inhalable substance to a consumer, and a wall with an outer surface and an inner surface that defines an interior space. Various implementations of an aerosol source member (or cartridge) are described in U.S. Pat. No. 9,078,473 to Worm et al., which is incorporated herein by reference in its entirety.

Although some figures described herein illustrate the control body and aerosol source member in a working relationship, it is understood that the control body and the aerosol source member may exist as individual devices. Accordingly, any discussion otherwise provided herein in relation to the components in combination also should be understood as applying to the control body and the aerosol source member as individual and separate components.

In another aspect, the present disclosure may be directed to kits that provide a variety of components as described herein. For example, a kit may comprise a control body with one or more aerosol source members. A kit may further comprise a control body with one or more charging components. A kit may further comprise a control body with one or more batteries. A kit may further comprise a control body with one or more aerosol source members and one or more charging components and/or one or more batteries. In further implementations, a kit may comprise a plurality of aerosol source members. A kit may further comprise a plurality of aerosol source members and one or more batteries and/or one or more charging components. In the above implementations, the aerosol source members or the control bodies may be provided with a heating member inclusive thereto. The inventive kits may further include a case (or other packaging, carrying, or storage component) that accommodates one or more of the further kit components. The case could be a reusable hard or soft container. Further, the case could be simply a box or other packaging structure.

Many modifications and other embodiments of the disclosure will come to mind to one skilled in the art to which this disclosure pertains having the benefit of the teachings presented in the foregoing descriptions and the associated drawings. Therefore, it is to be understood that the disclosure is not to be limited to the specific embodiments disclosed herein and that modifications and other embodiments are intended to be included within the scope of the appended claims. Although specific terms are employed herein, they are used in a generic and descriptive sense only and not for purposes of limitation.

### Statements:

1. An aerosol source member comprising:
   a substrate portion that includes a substrate material configured to generate a primary inhalable substance upon the application of heat thereupon, the substrate portion defining an outer surface; and
   an overwrap material configured to substantially surround the outer surface of the substrate portion,
   wherein the overwrap material comprises a plurality of layers, and wherein at least one of the layers of the overwrap material contains an aerosol generating component.
2. The aerosol source member of Statement 1, wherein the overwrap material is configured to produce an initial inhalable substance prior to production of a primary inhalable substance from the substrate material.
3. The aerosol source member of Statement 1, wherein the overwrap material is configured to produce a secondary inhalable substance after production of a primary inhalable substance from the substrate material.
4. An overwrap material configured to substantially surround an outer surface of a substrate portion of an aerosol source member, the overwrap material comprising:
   a plurality of layers, wherein at least one of the layers of the overwrap material contains an aerosol generating component.
5. The aerosol source member of Statement 1 or the overwrap material of Statement 4, wherein the aerosol generating component contained in the overwrap material has an aerosol former loading of approximately 2% - 20%.
6. The aerosol source member of Statement 1 or the overwrap material of Statement 4, wherein the aerosol generating component contained in the overwrap material has an aerosol former loading of approximately 20% - 40%.
7. The aerosol source member of Statement 1 or the overwrap material of Statement 4, wherein the overwrap material comprises a three layer laminate that includes an outer layer, an inner layer positioned proximate an outer surface of the substrate material, and an intermediate layer positioned between the outer layer and the inner layer.
8. The aerosol source member of Statement 7 or the overwrap material of Statement 7, wherein the outer layer comprises a paper material, the intermediate layer comprises a foil material, and the inner layer comprises an aerosol generating material.
9. The aerosol source member of Statement 8 or the overwrap material of Statement 8, wherein the aerosol generating material comprises a cast sheet containing a fibrous material.
10. The aerosol source member of Statement 8 or the overwrap material of Statement 8, wherein the aerosol generating material comprises a reconstituted tobacco sheet.
11. The aerosol source member of Statement 1 or the overwrap material of Statement 4, wherein the overwrap material is configured to produce the initial inhalable substance in response to application of heat from an electrically generated heat source.
12. The aerosol source member of Statement 1 or the overwrap material of Statement 4, wherein the overwrap material is configured to produce the initial inhalable substance in response to application of heat from a combustible carbon-based heat source.

## Claims

1. An overwrap material configured to substantially surround an outer surface of a substrate portion of an aerosol source member, the overwrap material comprising:
a plurality of layers, wherein at least one of the layers of the overwrap material contains an aerosol generating component, and wherein upon the application of heat thereupon, the overwrap material is configured to produce an inhalable substance,
wherein the overwrap material comprises at least an outer layer, an inner layer positioned proximate an outer surface of the substrate material, and an intermediate layer positioned between the outer layer and the inner layer, wherein the outer layer comprises a paper material, the intermediate layer comprises a foil material, and the inner layer comprises an aerosol generating material.

2. The overwrap material of Claim 1, wherein the aerosol generating component contained in the overwrap material has an aerosol former loading of approximately 2% - 20%.

3. The overwrap material of Claim 1, wherein the aerosol generating component contained in the overwrap material has an aerosol former loading of approximately 20% - 40%.

4. The overwrap material of any of Claims 1-3, wherein the aerosol generating material comprises a cast sheet containing a fibrous material.

5. The overwrap material of any of Claims 1-3, wherein the aerosol generating material comprises a reconstituted tobacco sheet.

6. The overwrap material of any of Claims 1-5, wherein the overwrap material is configured to produce the inhalable substance in response to application of heat from an electrically generated heat source.

7. The overwrap material of any of Claims 1-5, wherein the overwrap material is configured to produce the inhalable substance in response to application of heat from a combustible carbon-based heat source.

8. The overwrap material of any of Claim 1-7, wherein the overwrap material is configured to produce the inhalable substance in response to application of heat from inside of an aerosol source member containing the overwrap material.

9. The overwrap material of any of Claim 1-7, wherein the overwrap material is configured to produce the inhalable substance in response to application of heat from outside of an aerosol source member containing the overwrap material.

10. The overwrap material of any of Claims 1-5 and 7 further comprising a plurality of cooling openings formed in the overwrap material.

11. The overwrap material of Claim 10, wherein the cooling openings are formed in an area proximate a mouthend of the overwrap material.

12. The overwrap material of any of Claims 10-11, wherein the cooling openings comprise a plurality of openings substantially evenly spaced around the outer surface of the overwrap material.

13. The overwrap material of any of Claims 1-5 and 7 further comprising a plurality of air entry openings.

14. The overwrap material of Claim 13, wherein the plurality of air entry openings are formed in an area proximate a heat source end of the overwrap material.

15. The overwrap material of any of Claims 10-14, wherein the plurality of cooling openings and/or the plurality of air entry openings are formed via laser perforation.
